# EUROPEAN PATENT SPECIFICATION

(11) **EP 4 609 867 B1**
(45) Date of publication and mention of the grant of the patent: **02.09.2026**
(21) Application number: 23888076.9
(22) Date of filing: 09.11.2023
(51) Int. Cl.: A61K 9/51, C12N 15/88, A61K 31/7088, A61P 35/00

(54) **LIPID-CHARGED MOLECULE CONJUGATE, INHALABLE LIPID NANOPARTICLE, PREPARATION METHOD THEREFOR, AND USE THEREOF**
LIPIDBELADENES MOLEKÜLKONJUGAT, INHALIERBARES LIPIDNANOPARTIKEL, HERSTELLUNGSVERFAHREN DAFÜR UND VERWENDUNG DAVON
CONJUGUÉ DE MOLÉCULE CHARGÉE DE LIPIDES, NANOPARTICULE LIPIDIQUE INHALABLE, PROCÉDÉ DE PRÉPARATION S'Y RAPPORTANT ET UTILISATION ASSOCIÉE

(30) Priority: 10.11.2022 CN 202211406727
(43) Date of publication of application: 03.09.2025
(73) Proprietor: INSTITUTE OF CHEMISTRY, CHINESE ACADEMY OF SCIENCES, Beijing 100190 (CN)
(72) Inventor: LU, Xueguang, Beijing 100190 (CN); LIU, Shuai, Beijing 100190 (CN)
(74) Representative: Gille Hrabal Partnerschaftsgesellschaft mbB Patentanwälte
(86) International application number: PCT/CN2023/130743
(87) International publication number: WO 2024/099391

(56) References cited:
- EP-A1- 3 988 089
- WO-A1-2017/201347
- US-A1- 2018 078 625
- US-A1- 2022 249 704
- QIN J. ET AL.: "RGD peptide-based lipids for targeted mRNA delivery and gene editing applications", vol. 12, no. 39, 1 January 2022 (2022-01-01), GB, pages 25397 - 25404, XP093335180, ISSN: 2046-2069, Retrieved from the Internet <URL:http://pubs.rsc.org/en/content/articlepdf/2022/RA/D2RA02771B> DOI: 10.1039/D2RA02771B
- HIRAI Y. ET AL.: "Charge-reversible lipid derivative: A novel type of pH-responsive lipid for nanoparticle-mediated siRNA delivery", INTERNATIONAL JOURNAL OF PHARMACEUTICS, ELSEVIER, AMSTERDAM, NL, vol. 585, 27 May 2020 (2020-05-27), XP086208599, ISSN: 0378-5173, [retrieved on 20200527], DOI: 10.1016/J.IJPHARM.2020.119479
- LIU CHUNYAN, ZHAO WENLI; ZHANG LIGANG; SUN HUAMIN; CHEN XI; DENG NING: "Preparation of DSPE-PEG-cRGD Modified Cationic Liposomes for Delivery of OC-2 shRNA and The Antitumor Effects on Breast Cancer", PHARMACEUTICS, MDPI AG, SWITZERLAND, vol. 14, no. 10, Switzerland, pages 2157, XP093170772, ISSN: 1999-4923, DOI: 10.3390/pharmaceutics14102157
- HIRAI YUSUKE; SAEKI RYOKO; SONG FURAN; KOIDE HIROYUKI; FUKATA NAOFUMI; TOMITA KOJI; MAEDA NORIYUKI; OKU NAOTO; ASAI TOMOHIRO: "Charge-reversible lipid derivative: A novel type of pH-responsive lipid for nanoparticle-mediated siRNA delivery", INTERNATIONAL JOURNAL OF PHARMACEUTICS, ELSEVIER, NL, vol. 585, 27 May 2020 (2020-05-27), NL , XP086208599, ISSN: 0378-5173, DOI: 10.1016/j.ijpharm.2020.119479

## Description

### Technical Field

The present disclosure relates to the technical field of biomedicine, and particularly to a lipid-charged molecule conjugate, an inhalable lipid nanoparticle, and use thereof.

### Background

Messenger ribonucleic acid (mRNA), as a class of nucleic acid drugs, can be used to prepare a wide range of vaccines. Firstly, compared with conventional protein- and virus-based vaccines, mRNA can express different antigens in transfected cells only by changing the nucleotide sequence, and can be used to prepare vaccines for almost all infectious diseases or cancers. Secondly, mRNA is synthesized by an *in vitro* transcription method, which is simple, has a high yield, and can be used for rapid scale-up production without the need to purify virus, greatly shortening the research, development, and production time of vaccines. Finally, mRNA can transiently express proteins in cytoplasm without entering nucleus, so there is no risk of damaging genomes or causing gene mutations, and it is safer than plasmid DNA vaccines. However, mRNA has a high molecular weight and is negatively charged, so it is difficult to enter cells. Moreover, mRNA has a poor stability and is prone to be degraded by nucleases. Therefore, a suitable delivery vector is required to assist its transfection into target cells.

The outbreak of COVID-19 pandemic has led to a record-breaking approval and marketing of COVID-19 mRNA vaccines developed by Modern and Pfizer/BioNTech. Both of the approved mRNA vaccines use lipid nanoparticles as delivery vectors, and are administered via intramuscular injection to generate a systemic immune response. However, a vast majority of infectious viruses enter bodies through mucosal sites (mainly including mucosal sites of respiratory tract, gastrointestinal tract, reproductive system and the like). Although immunization via intramuscular injection can generate systemic antibody and cellular immune responses, which kill or neutralize pathogens that have already entered the blood stream, it cannot prevent the survival of pathogens on the mucosal surface, so that infected individuals after vaccination can still continue to spread the pathogens as a host. Therefore, only by activating the mucosal immune response, the pathogens on the mucosal surface can be effectively eliminated, so that the infection and spread of pathogens can be blocked at the source.

Directly delivering mRNA vaccines to nasal cavity, trachea, and lung by inhalation administration can simulate the natural infection process of pathogens and directly activate the mucosal immune response. Respiratory tract immunization has a series of advantages such as a low antigen dosage, a reduced risk of immune tolerance, avoidance of first-pass effect in livers, convenient administration, and good patient compliance, and thus has a great potential in clinical application. However, additional challenges are brought to inhalable mRNA vaccines due to the physiological characteristics of respiratory tract. Lipid nanoparticles prepared by self-assembly are extremely prone to rupture due to the influence of aerodynamic force and liquid surface tension during the process of nebulization to form an aerosol, and thus have poor stability. Further, they have a low efficiency of passing through the mucus layer on the surface of the mucous membrane, thus cannot effectively activate the immune cells in the respiratory tract. Therefore, only by improving the stability of lipid nanoparticles during nebulization in the process of the inhalation administration can a further clinical use of inhalable mRNA vaccines be realized.

### Summary

In order to solve the problems of instability during nebulization and low mRNA expression efficiency after inhalation administration of existing lipid nanoparticles, the technical solutions of the present disclosure are as follows:
Provided is a lipid-charged molecule conjugate, comprising a lipid unit and a charged unit.

According to an embodiment of the present disclosure, the lipid-charged molecule conjugate is an amphiphilic molecule having a surface potential of -1 mV to -40 mV or 1 mV to 40 mV.

According to an embodiment of the present disclosure, the lipid-charged molecule conjugate can be positively charged or negatively charged, preferably negatively charged.

According to an embodiment of the present disclosure, the charged unit is a negatively charged unit and/or a positively charged unit.

According to an embodiment of the present disclosure, the negatively charged unit is a unit composed of at least one of the following negatively charged substances: a first polypeptide, a nucleic acid unit (DNA, RNA and the like), a negatively charged polymer, a small negatively charged molecule and the like.

Preferably, the first polypeptide is a hydrophilic peptide chain containing a negatively charged amino acid and having a length of 1 to 50 amino acids, wherein the negatively charged amino acid is, for example, aspartic acid, and glutamic acid. Exemplarily, the first polypeptide is aspartic acid-serine-serine-cysteine.

Preferably, the nucleic acid unit is a DNA or RNA of any sequence with a length of 1 to 40 bases.

Preferably, the negatively charged polymer is a hydrophilic polymer selected from hyaluronic acid, polyacrylic acid, a carboxylic acid-modified polyethylene glycol, a phosphoric acid-modified polyethylene glycol and the like.

Preferably, the small negatively charged molecule is selected from a small molecular compound with a carboxylic acid group, a small molecular compound with a phosphoric acid group, a small molecular compound with a sulfonic acid group, a small molecular compound with a nitric acid group, and the like. Preferably, the small negatively charged molecule is at least one of 2-(3-amino-3-oxopropyl)malonic acid, succinic acid, 2-aminobutanedioic acid, formic acid, acetic acid, propionic acid, butyric acid, valeric acid, caproic acid, heptanoic acid, caprylic acid, pelargonic acid, capric acid, undecanoic acid, lauric acid, tridecanoic acid, myristic acid, pentadecanoic acid, palmitic acid, margaric acid, stearic acid, retinoic acid, tamibarotene and the like.

According to an embodiment of the present disclosure, the positively charged unit is a unit composed of at least one of the following positively charged substances: a second polypeptide, a positively charged polymer and the like.

Preferably, the second polypeptide is a hydrophilic peptide chain containing a positively charged amino acid and having a length of 1 to 50 amino acids, wherein the positively charged amino acid is selected from histidine, arginine, lysine and the like. Preferably, the positively charged polymer is at least one cationic polymer selected from polylysine, chitosan, a poly(β-amino ester) and the like.

According to an embodiment of the present disclosure, the lipid unit comprises a hydrophobic group, wherein the hydrophobic group can be a hydrophobic group known in the art, and is not specifically limited in the present disclosure. Exemplarily, the hydrophobic group is a hydrophobic hydrocarbon chain, for example, a substituted or unsubstituted saturated hydrocarbon group (for example, a C₃ - C₂₀ saturated hydrocarbon group, for example, a C₁₀ - C₂₀ saturated hydrocarbon group) or a hydrocarbon group with an unsaturated bond (a C₃ - C₂₀ hydrocarbon group with an unsaturated bond, for example, a C₁₀ - C₂₀ hydrocarbon group with an unsaturated bond). In case of being substituted, the substituent is, for example, at least one of an aryl group, an ester group, an ether group, an amine group, an amide group and the like. Exemplarily, the unsaturated bond is, for example, a carbon-carbon double bond. The hydrophobic group constitutes the lipid end of the lipid-charged molecule conjugate, and provides a hydrophobic interaction for the lipid-charged molecule conjugate, so that the lipid-charged molecule conjugate can self-assemble with the lipid components described below through hydrophilic-hydrophobic interaction to form the lipid nanoparticle described below. That is, the lipid unit is not limited to a certain lipid structure, for example, it is not limited to the ionizable lipid, phospholipid, sterol compound, fatty acid and the like described below, as long as it has a molecular structure which can provide a hydrophobic interaction. For example, the lipid unit can be provided by a hydrophobic substituted or unsubstituted hydrocarbon, for example, a substituted or unsubstituted saturated hydrocarbon (for example, a C₃ - C₂₀ saturated hydrocarbon, for example, a C₁₀ - C₂₀ saturated hydrocarbon) or a hydrocarbon with an unsaturated bond (a C₃ - C₂₀ hydrocarbon with an unsaturated bond, for example, a C₁₀ - C₂₀ hydrocarbon with an unsaturated bond). In case of being substituted, the substituent is, for example, at least one of an aryl group, an ester group, an ether group, an amine group, an amide group and the like. Exemplarily, the lipid unit is provided by a C₁₅ alkane.

According to an embodiment of the present disclosure, the lipid unit is at least one of an ionizable lipid, a phospholipid, a sterol compound, a fatty acid and the like. In the present disclosure, the ionizable lipid, phospholipid, sterol compound, and fatty acid can be those known in the art, and are not specifically limited in the present disclosure.

Exemplarily, the ionizable lipid is at least one of heptadec-9-yl 8-[(2-hydroxyethyl)(6-oxo-6-undecyloxyhexyl)amino]octanoate (SM-102), [(4-hydroxybutyl)azanediyl]bis(hexane-6,1-diyl) bis(2-hexyldecanoate) (ALC-0315), (dilinoleyl) methyl 4-(N,N-dimethylamino)butyrate (DLin-MC3-DMA), 3,6-bis{4-[bis(2-hydroxydodecyl)amino]butyl}piperazine-2,5-dione (cKK-E12), bis((Z)-non-2-en-1-yl) 9-(4-(dimethylamino)butyryloxy)heptadecanedioate (L319), 2,2-dilinoleyl-4-(2-dimethylaminoethyl)-[1,3]-dioxolane (DLin-KC2-DMA), heptadec-9-yl 8-[(2-hydroxyethyl)(8-nonyloxy-8-oxooctyl)amino]octanoate (Lipid 5), 1,1'-[(2-{4-[2-({2-[bis(2-hydroxydodecyl)amino]ethyl}(2-hydroxydodecyl)amino)ethyl]piperazin-1 -yl}ethyl)azanediyl]bis(dodecan-2-ol) (C12-200), (2,3-dioleoyloxy-propyl)trimethylammonium chloride (DOTAP), dimethyldioctadecylammonium bromide (DDAB), tetra(8-methylnonyl) 3,3',3",3‴-{[(methylazanediyl)bis(propane-3,1-diyl)]bis(azanetriyl)}tetrapropionate (306Oi10); preferably heptadec-9-yl 8-[(2-hydroxyethyl)(6-oxo-6-undecyloxyhexyl)amino]octanoate (SM-102) or [(4-hydroxybutyl)azanediyl]bis(hexane-6,1-diyl) bis(2-hexyldecanoate) (ALC-0315).

Exemplarily, the phospholipid is one or more of 1,2-distearoyl-sn-glycero-3- phosphocholine (DSPC), 1,2-dioleoyl-sn-glycero-3-phosphocholine (DOPC), 1,2-dipalmitoyl-sn-glycero-3-phosphocholine (DPPC), 2-oleoyl-1-palmitoyl-sn-glycero-3-phosphocholine (POPC), 1,2-dioleoyl-sn-glycero-3-phosphoethanolamine (DOPE), 2-oleoyl-1-palmitoyl-sn-glycero-3-phosphoethanolamine (POPE), 1,2-distearoyl-sn-glycero-3-phosphoethanolamine (DSPE), and 1,2-dipalmitoyl-sn-glycero-3-phosphoethanolamine (DPPE).

Exemplarily, the sterol compound is one or more of β-sitosterol, cholestanol, cholestanone, cholesterol, cholestenone, 7β-hydroxycholesterol, and 7α-hydroxycholesterol.

Exemplarily, the fatty acid is selected from a saturated fatty acid or an unsaturated fatty acid. The saturated fatty acid comprises palmitic acid, stearic acid, lauric acid, myristic acid, arachidic acid, or the like. The unsaturated fatty acid comprises myristoleic acid, palmitoleic acid, rapeseed acid, linoleic acid, linolenic acid, oleic acid, arachidonic acid, or the like.

According to an exemplary embodiment of the present disclosure, the lipid-charged molecule conjugate comprises a polypeptide unit and a lipid unit, named as a polypeptide-lipid conjugate molecule. For example, the lipid-charged molecule conjugate has a structure as shown in formula (I):

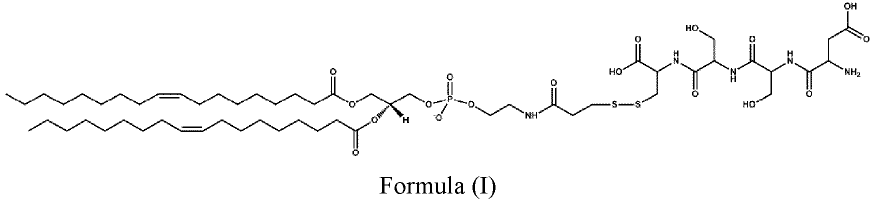

According to an exemplary embodiment of the present disclosure, the lipid-charged molecule conjugate is a small negatively charged molecule-lipid conjugate molecule, denoted as 2-hexyldecanoic acid, in which the small negatively charged molecule is formic acid, and the lipid unit is provided by pentadecane.

According to an exemplary embodiment of the present disclosure, the lipid-charged molecule conjugate is a DNA-lipid conjugate molecule, which is obtained by reacting azido-modified DOPE (DOPE-N₃) with dibenzocyclooctyne-modified adenine deoxyribonucleotide (DBCO-A). The DNA-lipid conjugate molecule has a structure as shown in formula (II):

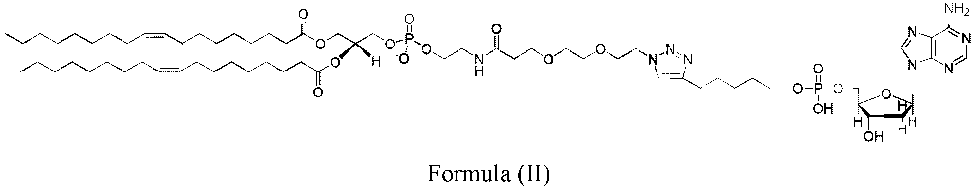

The present disclosure also provides a preparation method of the above lipid-charged molecule conjugate, comprising: using a lipid unit and a charged unit to prepare the lipid-charged molecule conjugate;

For example, a crosslinking agent is reacted with the lipid unit, then reacted with the charged unit to obtain the lipid-charged molecule conjugate.

According to an embodiment of the present disclosure, the lipid unit is prepared from at least one of an ionizable lipid, a phospholipid, a sterol compound, a fatty acid, wherein the ionizable lipid, phospholipid, sterol compound, and fatty acid have the meanings as described above.

Preferably, the lipid unit is obtained by reacting a phospholipid with a crosslinking agent.

Preferably, the crosslinking agent is succinimidyl 3-(2-pyridyldithio)propionate (SPDP).

According to an embodiment of the present disclosure, the molar ratio of the lipid unit to the charged unit is (0.8 - 1.2) : (0.8 - 1.2), for example, 1:1.

According to an exemplary embodiment of the present disclosure, the preparation method comprises: mixing the lipid unit with the crosslinking agent, removing the unreacted crosslinking agent after the reaction is completed, and then adding the negatively charged substance to obtain the lipid-charged molecule conjugate. Preferably, the molar ratio of the lipid unit to the crosslinking agent is (0.8 - 1.2) : (0.8 - 1.2), for example, 1:1. Preferably, the molar ratio of the lipid unit to the negatively charged unit is (0.8 - 1.2) : (0.8 - 1.2), for example, 1:1.

The present disclosure also provides use of the above lipid-charged molecule conjugate in the preparation of a lipid nanoparticle, a biological product or a pharmaceutical product. Preferably, the biological product is an inhalable biological product, such as an inhalable vaccine, and more preferably an inhalable mRNA vaccine.

The present disclosure also provides a lipid nanoparticle, which comprises the above lipid-charged molecule conjugate, lipid components, and a payload, wherein the lipid nanoparticle is formed through the self-assembly of the lipid-charged molecule conjugate with the lipid components and the payload via hydrophobic interaction and electrostatic interaction. In the present disclosure, the lipid-charged molecule conjugate is located on the surface of the lipid nanoparticle, which reduces the potential of the surface of the nanoparticle, enhances the electrostatic repulsion between the lipid nanoparticles, effectively reduces the collision and aggregation of the lipid nanoparticles, and thus improves the dispersion stability of the lipid nanoparticles in a solution. At the same time, on the same lipid nanoparticle, the electrostatic repulsion between the lipid-charged molecule conjugate molecules enhances the hydrophobic interaction between the lipid molecules, and further improves the stability of the lipid nanoparticle itself.

According to an embodiment of the present disclosure, by introducing the lipid-charged molecule conjugate on the surface of the lipid nanoparticle to regulate the electrostatic repulsion on the surface of the particles, the lipid nanoparticle has an improved stability during nebulization.

According to an embodiment of the present disclosure, the administration route of the lipid nanoparticle can be via an intravenous administration, a subcutaneous administration, an intramuscular administration, an inhalation administration, an intranasal administration, or the like, preferably an inhalation administration.

According to an embodiment of the present disclosure, by introducing the lipid-charged molecule conjugate into the lipid nanoparticle, the delivery efficiency of nucleic acid drugs after inhalation administration can be significantly improved.

According to an embodiment of the present disclosure, in the lipid nanoparticle, the lipid-charged molecule conjugate can be positively charged or negatively charged, preferably negatively charged.

According to an embodiment of the present disclosure, in the lipid nanoparticle, the molar ratio of the lipid-charged molecule conjugate to the lipid components is (0.1 - 20) : 100, preferably (0.6 - 10): 100, and preferably 2.5: 97.5.

According to an embodiment of the present disclosure, the molar ratio of the nitrogen content in the lipid components to the phosphorus content in the nucleic acid is (1 - 50):1; preferably (5 - 20):1, such as 1, 2, 3, 4, 5, 6, 7, 8, 9, or 10, and for example, 5.67.

According to an embodiment of the present disclosure, the lipid components comprise an ionizable lipid, a helper phospholipid, cholesterol and its derivatives, and a pegylated lipid.

According to an embodiment of the present disclosure, the molar percentage of the ionizable lipid with respect to the total amount of the lipid components is from 30% to 70%, such as 30%, 40%, 50%, 60%, or 70%.

According to an embodiment of the present disclosure, the ionizable lipid has the meaning as described above, and is, for example, at least one of heptadec-9-yl 8-[(2-hydroxyethyl)(6-oxo-6-undecyloxyhexyl)amino]octanoate (SM-102), [(4-hydroxybutyl)azanediyl]bis(hexane-6,1-diyl) bis(2-hexyldecanoate) (ALC-0315), (dilinoleyl) methyl 4-(N,N-dimethylamino)butyrate (DLin-MC3-DMA), 3,6-bis{4-[bis(2-hydroxydodecyl)amino]butyl}piperazine-2,5-dione (cKK-E12), bis((Z)-non-2-en-1-yl) 9-(4-(dimethylamino)butyryloxy)heptadecanedioate (L319), 2,2-dilinoleyl-4-(2-dimethylaminoethyl)-[1,3]-dioxolane (DLin-KC2-DMA), heptadec-9-yl 8-[(2-hydroxyethyl)(8-nonyloxy-8-oxooctyl)amino]octanoate (Lipid 5), 1,1'-[(2-{4-[2-({2-[bis(2-hydroxydodecyl)amino]ethyl}(2-hydroxydodecyl)amino)ethyl]piperazin-1 -yl}ethyl)azanediyl]bis(dodecan-2-ol) (C12-200), (2,3-dioleoyloxy-propyl)trimethylammonium chloride (DOTAP), dimethyldioctadecylammonium bromide (DDAB), tetra(8-methylnonyl) 3,3',3",3‴-{[(methylazanediyl)bis(propane-3,1-diyl)]bis(azanetriyl)}tetrapropionate (306Oi10); preferably heptadec-9-yl 8-[(2-hydroxyethyl)(6-oxo-6-undecyloxyhexyl)amino]octanoate (SM-102) or [(4-hydroxybutyl)azanediyl]bis(hexane-6,1-diyl) bis(2-hexyldecanoate) (ALC-0315).

According to an embodiment of the present disclosure, the helper phospholipid accounts for 0 mol% to 20 mol% of the total amount of the lipid components, such as 5 mol%, 6 mol%, 7 mol%, 8 mol%, 9 mol%, 10 mol%, 15 mol%, or 20 mol%.

According to an embodiment of the present disclosure, the helper phospholipid comprises but is not limited to one or more of 1,2-distearoyl-sn-glycero-3-phosphocholine (DSPC), 1,2-dioleoyl-sn-glycero-3-phosphocholine (DOPC), 1,2-dipalmitoyl-sn-glycero-3-phosphocholine (DPPC), 2-oleoyl-1-palmitoyl-sn-glycero-3-phosphocholine (POPC), 1,2-dioleoyl-sn-glycero-3-phosphoethanolamine (DOPE), 2-oleoyl-1-palmitoyl-sn-glycero-3-phosphoethanolamine (POPE), 1,2-distearoyl-sn-glycero-3-phosphoethanolamine (DSPE), and 1,2-dipalmitoyl-sn-glycero-3-phosphoethanolamine (DPPE), and is preferably DSPC.

According to an embodiment of the present disclosure, cholesterol and its derivatives account for 20 mol% to 50 mol% of the total amount of the lipid components, such as 20 mol%, 25 mol%, 30 mol%, 35 mol%, or 40 mol%, for example, 38.5 mol%.

According to an embodiment of the present disclosure, cholesterol and its derivatives comprise but are not limited to one or more of β-sitosterol, cholestanol, cholestanone, cholesterol, cholestenone, 7β-hydroxycholesterol, and 7α-hydroxycholesterol, and are preferably cholesterol.

According to an embodiment of the present disclosure, the pegylated lipid accounts for 0.1 mol% to 10 mol% of the total amount of the lipid components, preferably 1 mol% to 5 mol%, such as 1.0 mol%, 1.5 mol%, 2.0 mol%, 3.0 mol%, 4.0 mol%, or 5.0 mol%.

According to an embodiment of the present disclosure, the pegylated lipid comprises but is not limited to one or more of 1,2-dimyristoyl-rac-glycero-3-methoxypolyethylene glycol (DMG-PEG), 1,2-distearoyl-rac-glycero-3-methoxypolyethylene glycol (DSG-PEG), 1,2-dipalmitoyl-rac-glycero-3-methoxypolyethylene glycol (DPG-PEG), and 1,2-distearoyl-sn-glycero-3-phosphoethanolamine-methoxypolyethylene glycol (DSPE-PEG), and is preferably DMG-PEG.

According to an embodiment of the present disclosure, the payload is a nucleic acid, so as to achieve the delivery of the nucleic acid. Preferably, the nucleic acid is encapsulated in the lipid nanoparticle.

According to an embodiment of the present disclosure, the nucleic acid is at least one of a mRNA, a small interfering ribonucleic acid (siRNA), a plasmid, an antisense DNA, an aptamer, a microRNA, a circular RNA, and the like. Preferably, the mRNA is selected from, for example, mFLuc (a mRNA encoding firefly luciferase protein), S-Omicron (a mRNA encoding the spike protein of the Omicron variant of COVID-19), and mOVA (a mRNA encoding ovalbumin).

According to an embodiment of the present disclosure, the nucleic acid in the lipid nanoparticle has an encapsulation efficiency of 30% to 99%, preferably 70% to 90%, such as 70%, 75%, 80%, 85%, or 90%.

According to an embodiment of the present disclosure, the lipid nanoparticle has a hydrodynamic diameter of 50 to 200 nm, preferably 100 to 200 nm, for example, 120 to 180 nm, for example, 124 nm, 153 nm, 145 nm, or 151 nm.

In some embodiments, the molar ratio of the ionizable lipid : the helper phospholipid : cholesterol : the pegylated lipid in the lipid components is 50:10:38.5:1.5.

According to an exemplary embodiment of the present disclosure, the lipid nanoparticle comprises a lipid-charged molecule conjugate, lipid components, and an encapsulated nucleic acid, wherein
the lipid-charged molecule conjugate has a structure as shown in formula (I): 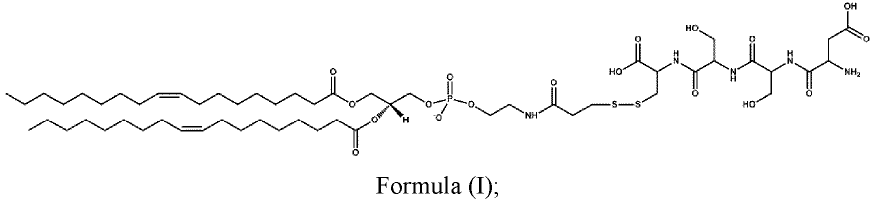
the lipid components are composed of SM102, DSPC, cholesterol and DMG-PEG2000 at a molar ratio of 50:10:38.5:1.5 or 50:9.4:38.5:1.5 or 50:7.5:38.5:1.5 or 50:0:38.5:1.5; and
the nucleic acid is mFLuc or mOVA.

The present disclosure also provides a preparation method for the above lipid nanoparticle, comprising mixing the nucleic acid, the lipid-charged molecule conjugate, and the lipid components, and then preparing the lipid nanoparticle by a microfluidic method.

According to an embodiment of the present disclosure, the microfluidic method comprises the following steps: taking the nucleic acid, the lipid-charged molecule conjugate and the lipid components, respectively, and injecting them into a microfluidic chip for mixing to obtain the lipid nanoparticle.

According to an embodiment of the present disclosure, the microfluidic method can be carried out by using a method known in the art. Exemplarily, an aqueous phase containing the nucleic acid and an organic phase containing the lipid-charged molecule conjugate and the lipid components are injected into a microfluidic chip for mixing at a flow ratio of 1:3, and the liquid collection is stopped until the aqueous phase solution in the syringe is emptied, thus obtaining the lipid nanoparticle.

The present disclosure also provides use of the above lipid nanoparticle in the preparation of a pharmaceutical preparation or a biological product.

According to an embodiment of the present disclosure, the biological product is an inhalable biological product, preferably, wherein it comprises the above lipid nanoparticle.

In some embodiments, the biological product is a vaccine, preferably an mRNA vaccine.

In some embodiments, the biological product can be an influenza vaccine, an AIDS (HIV) vaccine, a viral pneumonia (SARS, SARS-CoV-2, or the like) vaccine, a tuberculosis vaccine, a respiratory syncytial virus (RSV) vaccine, an enterovirus (such as EV71) vaccine, a lung cancer vaccine, or the like.

According to an embodiment of the present disclosure, the administration route of the biological product is via an inhalation administration.

According to an embodiment of the present disclosure, the biological product is used for preventing and/or treating infectious diseases transmitted through the mucous membrane, mucous membrane-related tumors, respiratory diseases, or the like. For example, the infectious diseases are selected from influenza, COVID-19, severe acute respiratory syndrome (SARS), pulmonary tuberculosis, bronchitis and pneumonia caused by RSV infection, AIDS, hand-foot-and-mouth disease caused by enterovirus infection, and the like. For example, the mucous membrane-related tumors can be oral cancer, lung cancer, or the like. For example, the respiratory diseases can be pulmonary fibrosis, bronchitis, asthma, pulmonary emphysema, bronchiectasis, byssinosis, allergic pneumonia, pleurisy, or the like.

The present disclosure also provides a pharmaceutical preparation or a biological product, wherein the biological product has the meaning as described above.

The present disclosure also provides a method for preventing and/or treating a disease, comprising administering a prophylactically and/or therapeutically effective amount of the above pharmaceutical preparation or biological product to a subject, wherein

the disease is infectious diseases transmitted through the mucous membrane, mucous membrane-related tumors, respiratory diseases, or the lime. For example, the infectious diseases are selected from influenza, COVID-19, severe acute respiratory syndrome (SARS), pulmonary tuberculosis, bronchitis and pneumonia caused by RSV infection, AIDS, hand-foot-and-mouth disease caused by enterovirus infection, and the like. For example, the mucous membrane-related tumors can be oral cancer, lung cancer, or the like. For example, the respiratory diseases can be pulmonary fibrosis, bronchitis, asthma, pulmonary emphysema, bronchiectasis, byssinosis, allergic pneumonia, pleurisy, or the like.

The above lipid nanoparticle, biological product and/or pharmaceutical preparation have any one, two or three of the following effects (1)-(3):
(1) The destructive effect caused by nebulization can be withstood, so as to deliver the lipid nanoparticle via nebulization inhalation, and then efficiently transfect the lung tissue cells and express the protein encoded by the mRNA.
(2) The mucosal immunity and systemic immunity can be effectively induced and generated.
(3) The antiviral immune protection and therapeutic efficacy for lung cancer can be effectively produced.

### Terminology Definitions and Explanations

The term "at least one" represents one, two or more than two.

The term "effective amount" refers to the amount of the biological product of the present disclosure that is sufficient to achieve the intended use (including but not limited to the treatment of diseases defined above), which can be determined according to the methods mastered by doctors with clinical practice qualification in the art. Determining the prophylactic and/or therapeutic effective dose is within the ability of clinical doctors or researchers in the art, and can be changed due to the following factors: the intended use (*in vitro* or *in vivo*), or the subject to be treated and the disease condition, such as the weight and age of the subject, general health status, severity of the disease condition, administration route, and other factors affecting the curative effect, such as the history of drug allergy. The specific administration dose will be changed depending on the following factors: the specific biological product selected, the dosage regimen followed, whether it is administered in combination with other drugs, the timing of administration, the tissue to which it is administered, and the physical delivery system it carries.

The "subject" in the present disclosure refers to a human or other warm-blooded mammal. The human as the "subject" in the present disclosure includes adults, infants, and children. Other warm-blooded mammal includes but is not limited to non-human primates, such as chimpanzees, other apes or monkeys, and other zoo animals, domestic mammals or laboratory animals, such as cats, pigs, dogs, cows, sheep, mice, rats, and guinea pigs.

### Beneficial effects

(1) The present disclosure provides a lipid-charged molecule conjugate and a method for improving the stability of a lipid nanoparticle. According to the present disclosure, the lipid-charged molecule conjugate is used to fabricate the lipid nanoparticle, increasing the charge on the surface of the lipid nanoparticle, and achieving the simultaneous enhancement of the hydrophobic interaction among lipid molecules and the electrostatic repulsion among lipid nanoparticles, thus significantly improving the stability of the lipid nanoparticle.
(2) The present disclosure provides a novel lipid nanoparticle for delivering nucleic acid drugs (such as mRNA, circular RNA, siRNA, microRNA, antisense nucleic acid, plasmid, or the like) via inhalation administration, a preparation method therefor, and use thereof. According to the present disclosure, by introducing a lipid-charged molecule conjugate to the lipid nanoparticle, the prepared novel lipid nanoparticle can resist the destructive effect of the inhalation delivery device during nebulization, so that the stability of the lipid nanoparticle during nebulization and thus the delivery efficiency of nucleic acid after inhalation administration can be significantly improved, and the nucleic acids (such as mRNA) can be efficiently expressed in the lung tissue, thereby solving the problem of the low inhalation delivery efficiency of existing lipid nanoparticles.
(3) The novel lipid nanoparticle of the present disclosure, after being administered via nebulization inhalation, can simultaneously activate humoral, cellular and mucosal immune responses, the intensity of which is significantly higher than that of the existing commercial lipid nanoparticle dosage forms, and thus has great application prospects in the field of preventing and treating infectious diseases transmitted through mucous membranes.

### Brief Description of the Drawings

FIG. 1 is a graph of the hydrodynamic diameter of different lipid nanoparticles in Test Example 1 of the present disclosure;
FIG. 2 is a graph of the ζ potential of different lipid nanoparticles in Test Example 2 of the present disclosure;
FIG. 3 is a graph of the encapsulation efficiency of different lipid nanoparticles before and after nebulization in Test Example 3 of the present disclosure;
FIG. 4 is a graph of the mRNA expression in mice after nebulization inhalation of different lipid nanoparticles in Application Example 1 of the present disclosure, in which (a) is a bioluminescence imaging graph of major organs *in vitro*; and (b) is a statistical result graph of the bioluminescence intensity of the lung tissue;
FIG. 5 is a graph of the proportion of tissue-resident memory T cells induced in the lung tissue of mice after nebulization inhalation of different lipid nanoparticles in Application Example 2 of the present disclosure, in which (a) is a scatter plot of the flow cytometry experiment; and (b) is a statistical result graph of the proportion of tissue-resident memory T cells;
FIG. 6 is a graph of the quantity of antigen-specific T cells generated in the spleen of mice after nebulization inhalation of different lipid nanoparticles in Application Example 2 of the present disclosure, in which (a) is an optical photograph of the ELISpot well; and (b) is a statistical result graph of the number of spots in the ELISpot well.
FIG. 7 is a graph of the prophylactic efficacy results of lung metastases after nebulization inhalation of different lipid nanoparticles in Application Example 2 of the present disclosure, in which (a) is an optical photograph of the lung tissue; and (b) is a statistical result graph of the number of metastatic tumor foci on the lung tissue;
FIG. 8 shows the content of antibody generated after nebulization inhalation of different lipid nanoparticles in Application Example 3, in which (a) shows the content of antigen-specific immunoglobulin G in the serum; (b) shows the content of neutralizing antibodies in the serum; (c) shows the content of antigen-specific immunoglobulin A in the bronchoalveolar lavage fluid (BALF); and (d) shows the content of neutralizing antibodies in the bronchoalveolar lavage fluid;
FIG. 9 is a mass spectrometry spectrum of pyridyldithio-modified 1,2-dioleoyl-sn-glycero-3-phosphoethanolamine (DOPE-PDP) in Preparation Example 1;
FIG. 10 is a mass spectrometry spectrum of the polypeptide-lipid conjugate molecule (DOPE-DSSC) in Preparation Example 1;
FIG. 11 is a graph of the hydrodynamic diameter of the lipid nanoparticle in Test Example 4 of the present disclosure;
FIG. 12 is a graph of the ζ potential of the lipid nanoparticle in Test Example 5 of the present disclosure;
FIG. 13 is a graph of the encapsulation efficiency of the lipid nanoparticle before and after nebulization in Test Example 6 of the present disclosure;
FIG. 14 is a graph of the mRNA expression in mice after nebulization inhalation of different lipid nanoparticles in Application Example 4 of the present disclosure, in which (a) is a bioluminescence imaging graph of the various organs *in vitro*; and (b) is a statistical result graph of the bioluminescence intensity of the lung tissue; and
FIG. 15 is a graph of the encapsulation efficiency of the lipid nanoparticles before and after nebulization in Test Example 7 of the present disclosure.

### Detailed Description

The technical solutions of the present disclosure will be further described in detail below in combination with the specific examples. It should be understood that the following examples are only for exemplarily illustrating and explaining the present disclosure, and should not be construed as limiting the protection scope of the present disclosure. All technologies implemented based on the above content of the present disclosure are covered within the scope intended to be protected by the present disclosure.

Unless otherwise specified, the raw materials and reagents used in the following examples are commercially available products or can be prepared by known methods.

### Preparation Example 1

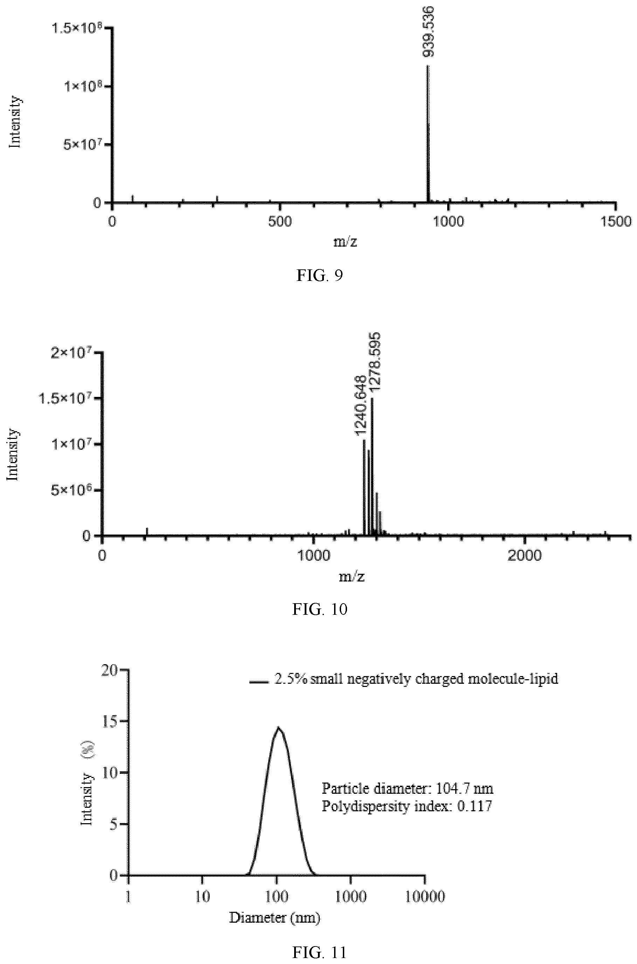

The polypeptide-lipid conjugate molecule shown in formula (I) was prepared according to the following steps:
(1) Equimolar 1,2-dioleoyl-sn-glycero-3-phosphoethanolamine (DOPE) and succinimidyl 3-(2-pyridyldithio)propionate (SPDP) were dissolved in dichloromethane, to which five equivalents of triethylamine were added, and then reacted overnight at room temperature. The obtained reaction mixture was separated by a silica gel column chromatography to yield pyridyldithio-modified 1,2-dioleoyl-sn-glycero-3-phosphoethanolamine (DOPE-PDP). The mass spectrometry characterization results of the DOPE-PDP molecule were shown in FIG. 9.
(2) Equimolar DOPE-PDP and a negatively charged polypeptide containing a sulfhydryl group (aspartic acid-serine-serine-cysteine, DSSC) were dissolved in dimethyl sulfoxide, and reacted overnight at room temperature. The obtained reaction mixture was dialyzed in water to remove dimethyl sulfoxide, and extracted with water and ethanol respectively to remove the unreacted DSSC and DOPE-PDP, thus yielding a purified DSSC-DOPE conjugate, i.e., the polypeptide-lipid conjugate molecule shown in formula (I). The mass spectrometry characterization results of the polypeptide-lipid conjugate molecule were shown in FIG. 10.

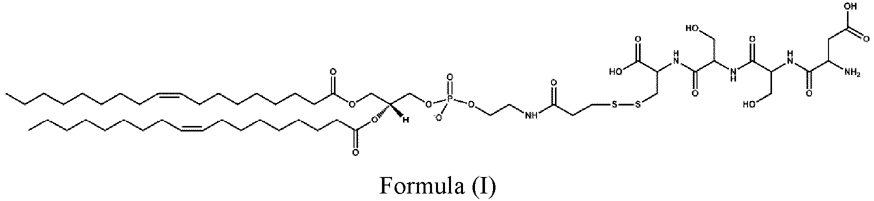

### Examples 1-3

The inhalable lipid nanoparticles were prepared as follows:
The inhalable lipid nanoparticles of these examples were prepared according to the preparation method of the mRNA-1273 formulation in Comparative Example 1. The difference was that: DSPC in the clinically used lipid nanoparticle of Comparative Example 1 was completely or partially replaced with the polypeptide-lipid conjugate molecule. Specifically, the polypeptide-lipid conjugate molecules in Examples 1, 2, and 3 were 0.6 mol%, 2.5 mol%, and 10 mol% of the total amount of SM102, DSPC, cholesterol, DMG-PEG2000 and the polypeptide-lipid conjugate molecule, respectively. The remaining conditions were the same as those of the mRNA-1273 formulation in Comparative Example 1. Then, inhalable lipid nanoparticles containing different proportions of the polypeptide-lipid conjugate molecule were prepared by a microfluidic method, named as 0.6% polypeptide-lipid, 2.5% polypeptide-lipid, and 10% polypeptide-lipid, respectively.

### Comparative Example 1

The clinically used lipid nanoparticle was prepared as follows:
The formulation of the COVID-19 mRNA-1273 from Moderna Company was used as the clinically used lipid nanoparticle, which comprised 1-octylnonyl 8-[(2-hydroxyethyl)[6-oxo-6-(undecyloxy) hexyl]amino]octanoate (SM-102), distearoylphosphatidylcholine (DSPC), cholesterol, dimyristoyl glycerol-polyethylene glycol 2000 (DMG-PEG2000), and mRNA, wherein the molar ratio of SM102 : DSPC : cholesterol : DMG-PEG2000 was 50:10:38.5:1.5, and the nitrogen-phosphorus ratio of SM102 to mRNA was 5.67. The clinically used lipid nanoparticle of this comparative example was prepared by a microfluidic method, wherein mFluc, a mRNA encoding firefly luciferase, was used as the mRNA.

### Test Example 1: Hydrodynamic Diameters of the Clinically Used Lipid Nanoparticle and the Inhalable Lipid Nanoparticles

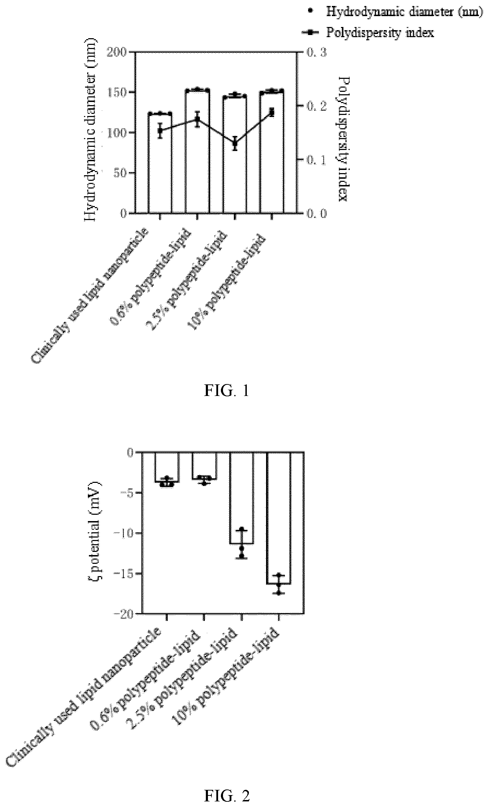

The clinically used lipid nanoparticle of Comparative Example 1 and the three inhalable lipid nanoparticles (0.6% polypeptide-lipid, 2.5% polypeptide-lipid, and 10% polypeptide-lipid) of Examples 1-3 were added into a phosphate buffer saline (PBS buffer) to obtain four PBS solutions containing 20 ng/µL mRNA. 60 µL of each of the above PBS solutions containing 20 ng/µL mRNA was tested using a dynamic light scattering method to measure the hydrodynamic diameters of the four different lipid nanoparticles in the PBS buffer. The results were shown in FIG. 1.

It can be seen from FIG. 1 that the above four different lipid nanoparticles can all form stable nanostructures in the PBS buffer. The clinically used lipid nanoparticle has an average hydrodynamic diameter of 124 nm, and 0.6% polypeptide-lipid, 2.5% polypeptide-lipid, and 10% polypeptide-lipid had average hydrodynamic diameters of 153 nm, 145 nm, and 151 nm, respectively. The polydispersity indexes of the particle sizes of the above four different lipid nanoparticles were all below 0.2, indicating that the formed nanoparticles had relatively uniform sizes.

### Test Example 2: ζ Potentials of the Clinically Used Lipid Nanoparticle and the Inhalable Lipid Nanoparticles

The clinically used lipid nanoparticle prepared in Comparative Example 1 and the three inhalable lipid nanoparticles of Examples 1-3 were added into a PBS buffer to obtain four PBS solutions containing 4 ng/µL mRNA. 500 µL of each of the above PBS solutions containing 4 ng/µL mRNA was tested using Zetasizer Nano ZSP instrument to measure the ζ potentials of the four lipid nanoparticles. The results were shown in FIG. 2.

It can be seen from FIG. 2 that the clinically used lipid nanoparticle had a ζ potential of -3.7 mV, and 0.6% polypeptide-lipid had essentially the same ζ potential as the clinically used lipid nanoparticle. Since a negatively charged polypeptide was used at the polypeptide end in the polypeptide-lipid conjugate molecule, the surface charge of the lipid nanoparticle was gradually decreased with the increase of the proportion of the polypeptide-lipid component. The ζ potentials of 2.5% polypeptide-lipid and 10% polypeptide-lipid were decreased to -11.4 mV and -16.3 mV respectively.

### Test Example 3: Encapsulation efficiencies of the Clinically Used Lipid Nanoparticle and the Inhalable Lipid Nanoparticles before and after nebulization

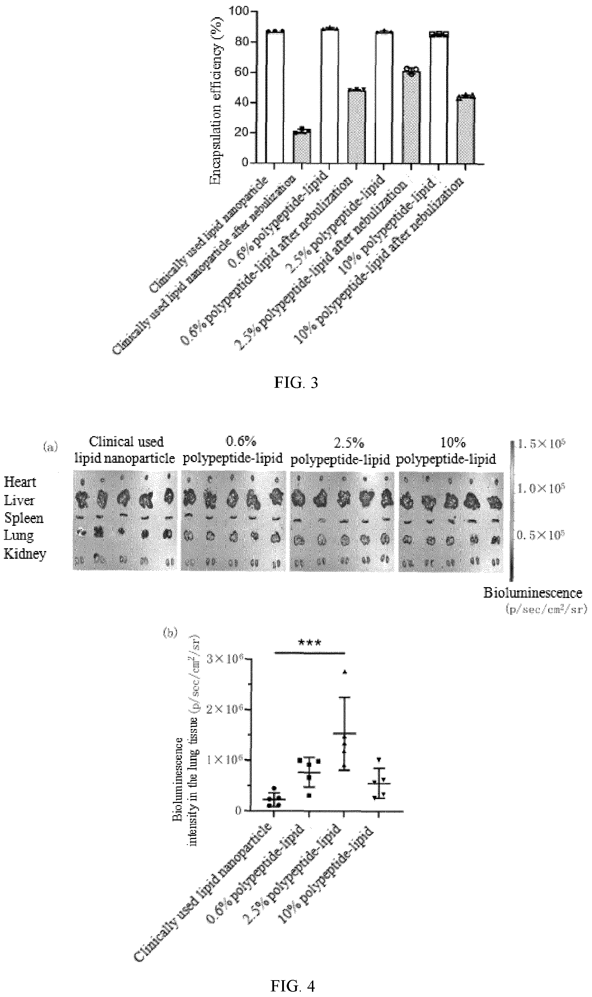

The clinically used lipid nanoparticle prepared in Comparative Example 1 and the three inhalable lipid nanoparticles prepared in Examples 1-3 were added into a PBS solution to obtain four solutions containing 25 ng/µL mRNA. 400 µL of each of these solutions was added to a clinically used nebulizer system Aerogen Solo to nebulize the four different lipid nanoparticles respectively. The mRNA encapsulation efficiencies of the four lipid nanoparticles before and after nebulization were detected with RiboGreen reagent and Triton X-100 membrane permeabilizing agent, so as to evaluate the degree of damage of the nebulizer system to the lipid nanoparticles. The results were shown in FIG. 3.

It can be seen from FIG. 3 that before nebulization, all of the four lipid nanoparticles had mRNA encapsulation efficiencies of 85% to 89%. After nebulization, the encapsulation efficiency of the clinically used lipid nanoparticle was decreased to 21%, and the encapsulation efficiencies of 0.6% polypeptide-lipid, 2.5% polypeptide-lipid, and 10% polypeptide-lipid were decreased to 48%, 62%, and 45% respectively. Thus, it can be seen that the three inhalable lipid nanoparticles prepared in Examples 1-3 had a better stability during nebulization than the clinically used lipid nanoparticle in Comparative Example 1, and the formulation containing 2.5% polypeptide-lipid had the best stability during nebulization.

### Application Example 1

### mRNA Expression Efficiencies in Mice after Nebulization Inhalation of Clinically Used Lipid Nanoparticle and the Inhalable Lipid Nanoparticles

Method: Female C57BL/6 mice aged 6-7 weeks were used as models to detect the mRNA expression efficiency *in vivo* after nebulization inhalation of the lipid nanoparticles.

The lipid nanoparticles prepared in Comparative Example 1 and Examples 1-3 were added into PBS buffers to formulate four solutions containing 20 ng/µL mFluc for later use.

The clinically used lipid nanoparticle prepared in Comparative Example 1 and the three inhalable lipid nanoparticles prepared in Examples 1-3 were nebulized using Aerogen Solo. 50 µL of each of the nebulized lipid nanoparticles containing 20 ng/µL mFluc was administered to each of the mice by tracheal inhalation. 24 hours after administration, each of the mice was intraperitoneally injected with 200 µL of a PBS solution containing 20 mg/mL D-luciferin potassium salt. 15 minutes later, the heart, liver, spleen, lung, and kidney of the mice were imaged by bioluminescence imaging respectively.

The test results were shown in FIG. 4. After nebulization inhalation of the four different lipid nanoparticles, the firefly luciferase encoded by mFluc was expressed only in the lung tissue of the mice. The clinically used lipid nanoparticle having the worst stability during nebulization had the lowest expression level in the lung tissue, while the 2.5% polypeptide-lipid having the best stability during nebulization had the highest expression level in the lung tissue, and its expression level in the lung tissue was 7 times that of the clinical lipid nanoparticle. The expression levels of 0.6% polypeptide-lipid and 10% polypeptide-lipid in the lung tissue were also 3.5 times and 2.5 times that of the clinically used lipid nanoparticle, respectively.

### Comparative Example 2

This comparative example was basically the same as Comparative Example 1, except that mFluc was replaced with a mRNA encoding ovalbumin (OVA), namely mOVA, thus obtaining a clinically used lipid nanoparticle.

### Example 4

The inhalable lipid nanoparticle in Example 4 was prepared according to Example 2, except that mFluc in Example 2 was replaced with mOVA, denoted as 2.5% polypeptide-lipid.

### Application Example 2

The clinically used lipid nanoparticle and the inhalable lipid nanoparticle were loaded with mRNA encoding a model antigen to evaluate the vaccine efficacy after nebulization inhalation.

Method: After nebulization inhalation of the clinically used lipid nanoparticle prepared in Comparative Example 2 and the inhalable lipid nanoparticle prepared in Example 4, the vaccine efficacies in a female C57BL/6 mouse model aged 6-7 weeks were evaluated.

The lipid nanoparticles prepared in Comparative Example 2 and Example 4 were added into PBS buffers to formulate two solutions containing 40 ng/µL mOVA for later use.

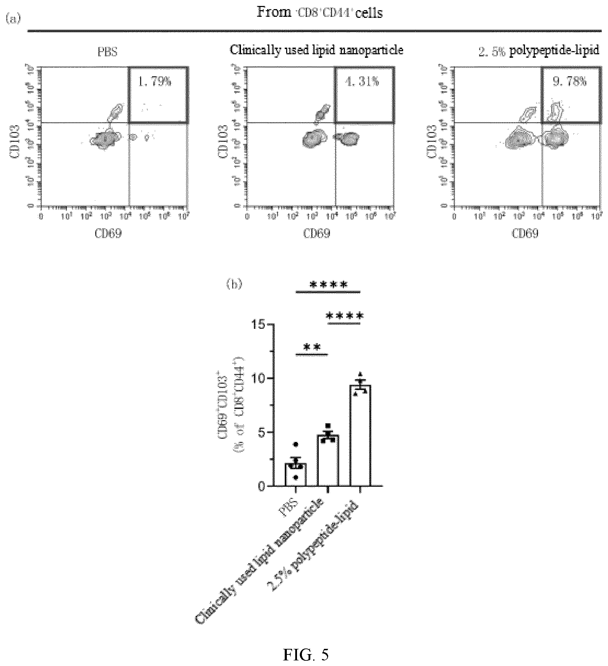

Female C57BL/6 mice aged 6-7 weeks were administered, via tracheal inhalation, with 50 µL of the nebulized clinically used lipid nanoparticle or 2.5% polypeptide-lipid containing 40 ng/µL mOVA each time on day 0 and day 14. In addition, mice were administered with 50 µL of PBS as a blank control group. On day 21 after first administration, the lung of the mouse was excised and prepared into a single-cell suspension. CD8, CD44, CD69 and CD103 were labelled with antibodies modified with different fluorescences respectively. Further, the percentage of CD69⁺ and CD103⁺ T cells of CD8⁺ and CD44⁺ T cells was detected by flow cytometry, so as to reflect the proportion of tissue-resident memory T cells in the lung tissue. The test results were shown in FIG. 5.

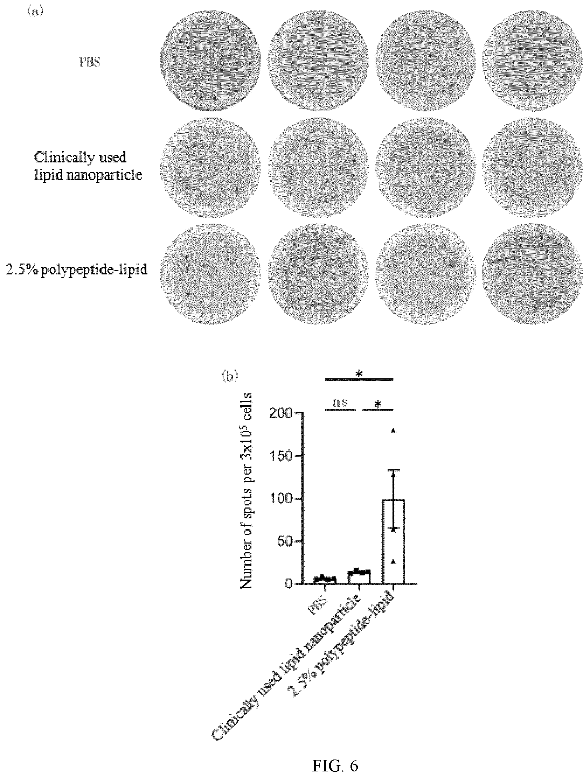

In addition, the spleen of the mouse was excised and prepared into a single-cell suspension. 300,000 cells were added to each well of an ELISpot plate for detecting IFN-γ-secreting cells, and then an 4 µg/mL of OVA antigen peptide was added to stimulate the splenocytes. After 24 hours of stimulation, the number of spots generated at the bottom of the ELISpot well was detected through a color development experiment, so as to reflect the number of antigen-specific T cells in the spleen. The test results were shown in FIG. 6.

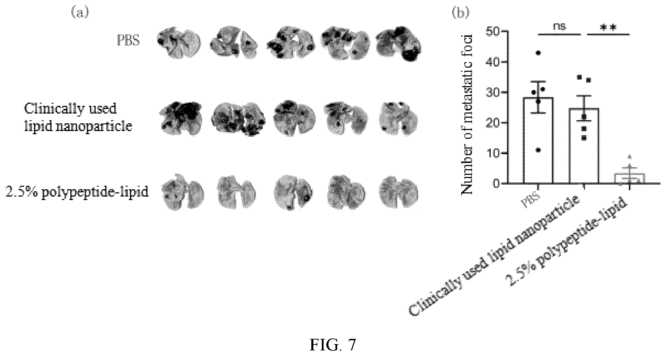

In order to verify the prophylactic effect of the clinically used lipid nanoparticle prepared in Comparative Example 2 and the inhalable lipid nanoparticle prepared in Example 4 on lung metastatic cancer after nebulization inhalation, a metastatic lung cancer model was established by injecting 200,000 mouse melanoma cells transfected with OVA via the tail vein on day 21 after the first administration. On day 22 after the establishment of the metastatic lung cancer model, the lung tissue of the mouse was dissected and taken out, and the number of melanoma foci on the lung tissue was counted. The results were shown in FIG. 7.

From the above test, it can be seen that for the clinically used lipid nanoparticle, the expression level of the antigen protein OVA in the lung tissue was low, and thus the vaccine efficacy was also low, since most of the clinically used lipid nanoparticles were damaged during nebulization. By contrast, after nebulization inhalation, the inhalable lipid nanoparticle (2.5% polypeptide-lipid) in Example 4 having a good stability during nebulization had a significantly higher vaccine efficacy than the clinically used lipid nanoparticle. The proportion of tissue-resident memory T cells induced by 2.5% polypeptide-lipid in the lung tissue was 2 times that of the clinically used lipid nanoparticle, and the number of antigen-specific T cells generated by 2.5% polypeptide-lipid in the spleen was 7 times that of the clinically used lipid nanoparticle. Moreover, 2.5% polypeptide-lipid can effectively inhibit the occurrence and development of metastatic lung cancer, while the clinically used lipid nanoparticle cannot inhibit metastatic lung cancer. Thus, it was proved that the inhalable lipid nanoparticles prepared according to the present disclosure can exert an excellent vaccine efficacy after nebulization inhalation and can provide an effective mucosal immune protection.

### Comparative Example 3

This comparative example was basically the same as Comparative Example 1, except that mFluc was replaced with a mRNA encoding the spike protein of the Omicron variant of COVID-19 (S-Omicron), namely mS-Omicron, thus obtaining a clinically used lipid nanoparticle.

### Example 5

The inhalable lipid nanoparticle in Example 5 was prepared according to Example 2, except that mFluc in Example 2 was replaced with mS-Omicron, denoted as 2.5% polypeptide-lipid.

### Application Example 3

The clinically used lipid nanoparticle and the inhalable lipid nanoparticle were loaded with a mRNA encoding the spike protein of the Omicron variant of COVID-19 to evaluate vaccine efficacy after nebulization inhalation.

Method: After nebulization inhalation of the clinically used lipid nanoparticle prepared in Comparative Example 3 and the inhalable lipid nanoparticle prepared in Example 5, the vaccine efficacy in a female C57BL/6 mouse model aged 6-7 weeks was evaluated.

The lipid nanoparticles prepared in Comparative Example 3 and Example 5 were added into PBS buffers to formulate solutions containing 40 ng/µL mS-Omicron for later use.

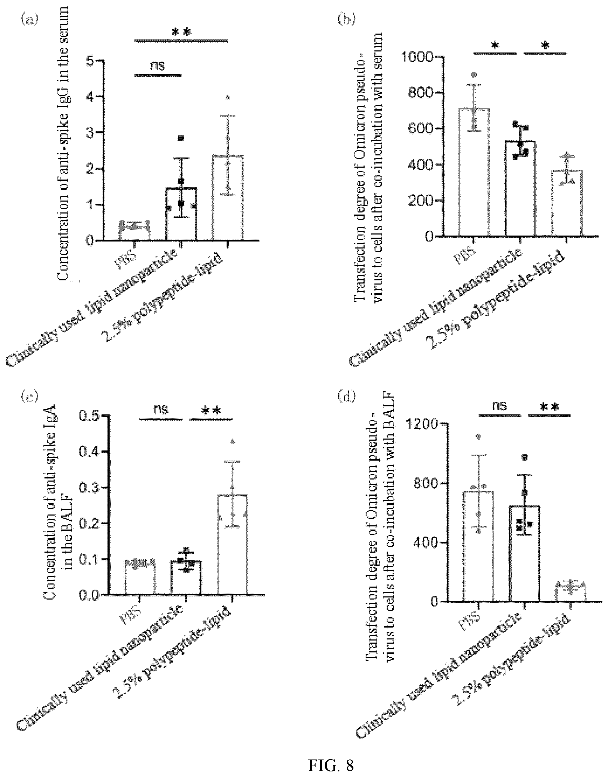

Female C57BL/6 mice aged 6-7 weeks were administered, by tracheal inhalation, with 50 µL of the nebulized clinically used lipid nanoparticle or 2.5% polypeptide-lipid containing 40 ng/µL mS-Omicron each time on day 0 and day 14. In addition, mice were administered with 50 µL of PBS as a blank control group. On day 21 after first administration, the serum and bronchoalveolar lavage fluid of the mice were collected. The concentration of immunoglobulin G specific for the spike protein of the Omicron variant of COVID-19 in the serum and the concentration of immunoglobulin A specific for the spike protein of the Omicron variant of COVID-19 in the bronchoalveolar lavage fluid were detected by enzyme-linked immunosorbent assay respectively, so as to reflect the systemic immunity and mucosal immunity generated after inhalation of the vaccine. The results were shown in (a) and (c) of FIG. 8.

In addition, the pseudovirus of the Omicron variant of COVID-19 was co-incubated with the serum and bronchoalveolar lavage fluid respectively, and then transfected into HEK-293T cells expressing angiotensin-converting enzyme 2. The transfection degree of the Omicron pseudovirus to these cells was detected to reflect the concentrations of neutralizing antibodies in the serum and bronchoalveolar lavage fluid. The results were shown in (b) and (d) of FIG. 8.

From the above test, it can be seen that for the clinically used lipid nanoparticle, the expression level of the antigen protein S-Omicron in the lung tissue was low, and low level of antibodies were produced, since most of the clinically used lipid nanoparticles were damaged during nebulization. By contrast, after nebulization inhalation, the inhalable lipid nanoparticle (2.5% polypeptide-lipid) in Example 5 having a good stability during nebulization produced significantly more antibodies than the clinically used lipid nanoparticle. Thus, it was proved that the inhalable lipid nanoparticle prepared according to the present disclosure can effectively induce the production of antigen-specific immunoglobulin G in the serum, antigen-specific immunoglobulin A in the bronchoalveolar lavage fluid and neutralizing antibodies after nebulization inhalation.

### Example 6

The inhalable lipid nanoparticle of this example was prepared according to the preparation method of the mRNA-1273 formulation in Comparative Example 1. The difference was that: a part of DSPC in the clinically used lipid nanoparticle of Comparative Example 1 was replaced with a small negatively charged molecule-lipid (2-hexyldecanoic acid, prepared from formic acid and pentadecane, purchased commercially), in which the small negatively charged molecule-lipid was 2.5 mol% of the total amount of SM102, DSPC, cholesterol, DMG-PEG2000 and the small negatively charged molecule-lipid. The remaining conditions are the same as those of the mRNA-1273 formulation in Comparative Example 1. Then, an inhalable lipid nanoparticle containing 2.5% small negatively charged molecule-lipid was prepared by a microfluidic method, denoted as 2.5% small negatively charged molecule-lipid.

### Test Example 4: Hydrodynamic Diameter of 2.5% Small Negatively Charged Molecule-Lipid

5% small negatively charged molecule-lipid of Example 6 was added to a PBS solution to obtain a PBS solution containing 20 ng/µL mRNA. 60 µL of the above solution was tested using the dynamic light scattering method to measure the hydrodynamic diameter of 2.5% small negatively charged molecule-lipid in the PBS buffer. The results were shown in FIG. 11. It can be seen from FIG. 11 that 2.5% small negatively charged molecule-lipid can form nanoparticles with uniform sizes.

### Test Example 5: ζ Potential of 2.5% Small Negatively Charged Molecule-Lipid

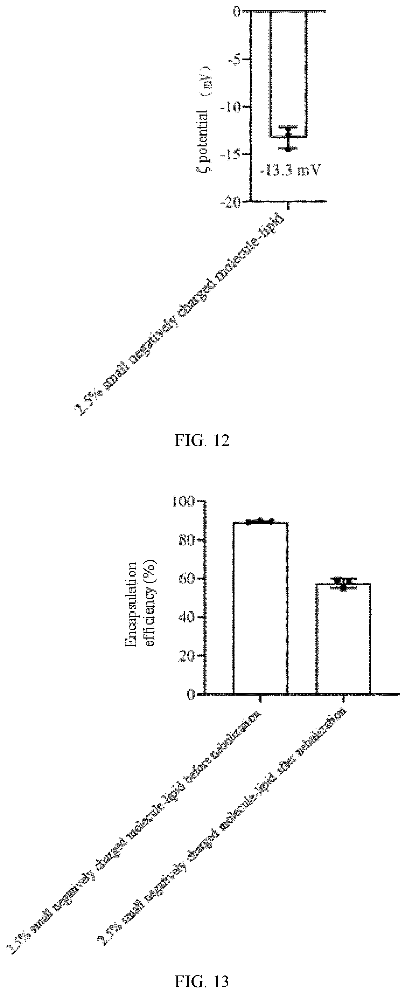

2.5% small negatively charged molecule-lipid of Example 6 was added into a PBS buffer to obtain a PBS solution containing 4 ng/µL mRNA. 500 µL of the above solution was tested using Zetasizer Nano ZSP instrument to measure the ζ potential. The results were shown in FIG. 12. It can be seen from FIG. 12 that 2.5% small negatively charged molecule-lipid had a ζ potential of -13.3 mV, indicating that the small negatively charged molecule-lipid can effectively impart a negative surface charge to the lipid nanoparticle.

### Test Example 6: Encapsulation Efficiency of 2.5% Small Negatively Charged Molecule-Lipid before and after Nebulization

2.5% small negatively charged molecule-lipid was added into a PBS solution to obtain a solution containing 25 ng/µL mRNA. 400 µL of this solution was added to a clinically used nebulizer system Aerogen Solo to nebulize it. The mRNA encapsulation efficiencies before and after nebulization were detected with RiboGreen reagent and Triton X-100 membrane permeabilizing agent, so as to evaluate the degree of damage of the nebulizer system to the lipid nanoparticle. The results were shown in FIG. 13. It can be seen from FIG. 13 that before nebulization, 2.5% small negatively charged molecule-lipid had an mRNA encapsulation efficiency of 89%. After nebulization, the encapsulation efficiency of 2.5% small negatively charged molecule-lipid still remained at 58%. Thus, it can be seen that 2.5% small negatively charged molecule-lipid still had a better stability during nebulization than the clinically used lipid nanoparticle in Comparative Example 1.

### Application Example 4

### mRNA Expression Efficiencies in Mice after Nebulization inhalation of the Clinically Used Lipid Nanoparticle and 2.5% Small Negatively Charged Molecule-Lipid

Method: Female C57BL/6 mice aged 6-7 weeks were used as models to detect the mRNA expression efficiencies *in vivo* after nebulization inhalation of the lipid nanoparticles.

The lipid nanoparticles prepared in Comparative Example 1 and Example 6 were added into PBS buffers to formulate two solution containing 20 ng/µL mFluc for later use.

The clinically used lipid nanoparticle prepared in Comparative Example 1 and 2.5% small negatively charged molecule-lipid prepared in Example 6 were nebulized using Aerogen Solo. 50 µL of each of the nebulized lipid nanoparticles containing 20 ng/µL mFluc was administered to each of the mice by tracheal inhalation. 24 hours after administration, each of the mice was intraperitoneally injected with 200 µL of a PBS solution containing 20 mg/mL D-luciferin potassium salt. 15 minutes later, the heart, liver, spleen, lung, and kidney of the mice were imaged by bioluminescence imaging respectively.

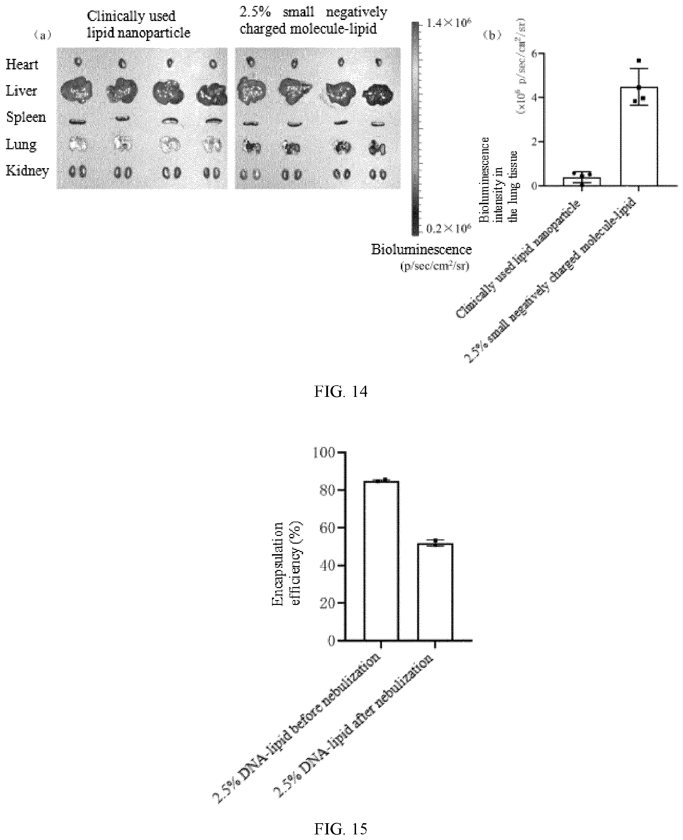

The test results were shown in FIG. 14. 2.5% small negatively charged molecule-lipid after nebulization inhalation had a significantly higher intensity of the firefly luciferase encoded by mFluc expressed in the mouse lung tissue than the clinically used lipid nanoparticle, indicating that 2.5% small negatively charged molecule-lipid having a good stability during nebulization can effectively transfect the lung tissue by delivering mRNA via inhalation.

### Preparation Example 2

The DNA-lipid conjugate molecule shown in formula (II) was prepared as follows:
(1) Equimolar 1,2-dioleoyl-sn-glycero-3-phosphoethanolamine (DOPE) and 2,5-dioxopyrrolidin-1-yl 3-(2-(2-(2-azidoethoxy)ethoxy)propionate were dissolved in dichloromethane, and reacted overnight at room temperature. The obtained reaction mixture was separated by a silica gel column chromatography to yield azido-modified DOPE (DOPE-N₃).
(2) Equimolar DOPE-N₃ and dibenzocyclooctyne-modified adenine deoxyribonucleotide (DBCO-A) were dissolved in dimethyl sulfoxide, and reacted overnight at room temperature. The obtained reaction mixture was dialyzed in water to remove dimethyl sulfoxide, and extracted with water and ethanol respectively to remove the unreacted DBCO-A and DOPE-N₃, thus yielding a purified DOPE-A conjugate, i.e., the DNA-lipid conjugate molecule shown in formula (II).

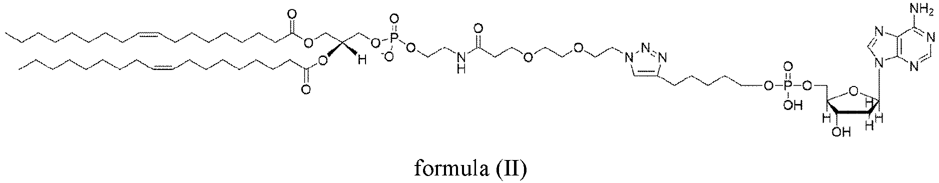

### Example 7

The inhalable lipid nanoparticle of this example was prepared according to the preparation method of the mRNA-1273 formulation in Comparative Example 1. The difference was that: a part of DSPC in the clinically used lipid nanoparticle of Comparative Example 1 was replaced with the DNA-lipid conjugate molecule of formula (II), in which the DNA-lipid conjugate molecule was 2.5 mol% of the total amount of SM102, DSPC, cholesterol, DMG-PEG2000 and the DNA-lipid conjugate molecule. The remaining conditions were the same as those of the mRNA-1273 formulation in Comparative Example 1. Then, an inhalable lipid nanoparticle containing 2.5% DNA-lipid conjugate molecule was prepared by a microfluidic method, denoted as 2.5% DNA-lipid.

### Test Example 7: Encapsulation Efficiencies of 2.5% DNA-lipid before and after Nebulization

2.5% DNA-lipid was added into a PBS solution to obtain a solution containing 25 ng/µL mRNA. 400 µL of this solution was added to a clinically used nebulizer system Aerogen Solo to nebulize it. The mRNA encapsulation efficiencies before and after nebulizationthe was detected with RiboGreen reagent and Triton X-100 membrane permeabilizing agent, so as to evaluate the degree of damage of the nebulizer system to the lipid nanoparticle. The results were shown in FIG. 15. It can be seen from FIG. 15 that before nebulization, 2.5% DNA-lipid had an mRNA encapsulation efficiency of 85%. After nebulization, the encapsulation efficiency of 2.5% DNA-lipid still remained at 52%. Thus, it can be seen that 2.5% DNA-lipid still had a better stability during nebulization than the clinically used lipid nanoparticle in Comparative Example 1.

If the mRNA in the above examples is replaced with a small interfering ribonucleic acid (siRNA), plasmid, antisense DNA, aptamer, microRNA, circular RNA, or the like, the inhalable lipid nanoparticles prepared also have basically the same effect as the inhalable lipid nanoparticles in the above examples.

## Claims

1. An inhalable lipid nanoparticle for use in preventing and/or treating infectious diseases transmitted through mucous membranes, mucous membrane-related tumors, or respiratory diseases, comprising a lipid-charged molecule conjugate, lipid components, and a payload, wherein the lipid-charged molecule conjugate is self-assembled with the lipid components and the payload by a hydrophobic interaction and an electrostatic interaction to form the lipid nanoparticle, wherein the lipid-charged molecule conjugate comprises a lipid unit and a charged unit, wherein the charged unit is a negatively charged unit and/or a positively charged unit;
wherein the inhalable lipid nanoparticle is administered by nebulization;
wherein the negatively charged unit is a unit composed of at least one of the following negatively charged substances: a first polypeptide, a nucleic acid unit, a negatively charged polymer, and a small negatively charged molecule, wherein the first polypeptide is a hydrophilic peptide chain containing a negatively charged amino acid and having a length of 1 - 50 amino acids; the positively charged unit is a unit composed of at least one of the following positively charged substances: a second polypeptide and a positively charged polymer, wherein the second polypeptide is a hydrophilic peptide chain containing a positively charged amino acid and having a length of 1 - 50 amino acids; and
the lipid unit comprises a hydrophobic group; and
wherein the negatively charged amino acid is aspartic acid or glutamic acid;
the nucleic acid unit is a DNA or RNA of any sequence with a length of 1 to 40 bases;
the negatively charged polymer is a hydrophilic polymer selected from hyaluronic acid, polyacrylic acid, a carboxylic acid-modified polyethylene glycol, or a phosphoric acid-modified polyethylene glycol;
the small negatively charged molecule is selected from a small molecular compound with a carboxylic acid group, a small molecular compound with a phosphoric acid group, a small molecular compound with a sulfonic acid group, and a small molecular compound with a nitric acid group;
the positively charged amino acid is selected from histidine, arginine, and lysine;
the positively charged polymer is at least one cationic polymer selected from polylysine, chitosan, and a poly(β-amino ester); and
the lipid unit comprises a substituted or unsubstituted saturated hydrocarbon group or a hydrocarbon group with an unsaturated bond.

2. The inhalable lipid nanoparticle for use according to claim 1, wherein the first polypeptide is selected from aspartic acid-serine-serine-cysteine; and
the small negatively charged molecule is at least one of 2-(3-amino-3-oxopropyl)malonic acid, succinic acid, 2-aminobutanedioic acid, formic acid, acetic acid, propionic acid, butyric acid, valeric acid, caproic acid, heptanoic acid, caprylic acid, pelargonic acid, capric acid, undecanoic acid, lauric acid, tridecanoic acid, myristic acid, pentadecanoic acid, palmitic acid, margaric acid, stearic acid, retinoic acid, or tamibarotene.

3. The inhalable lipid nanoparticle for use according to any one of claims 1 to 2, wherein the lipid unit is a unit composed of at least one of an ionizable lipid, a phospholipid, a sterol compound, and a fatty acid.

4. The inhalable lipid nanoparticle for use according to claim 3, wherein
the ionizable lipid is at least one of heptadec-9-yl 8-[(2-hydroxyethyl)(6-oxo-6-undecyloxyhexyl)amino]octanoate (SM-102), [(4-hydroxybutyl)azanediyl]bis(hexane-6,1-diyl) bis(2-hexyldecanoate) (ALC-0315), (dilinoleyl) methyl 4-(N,N-dimethylamino)butyrate (DLin-MC3-DMA), 3,6-bis{4-[bis(2-hydroxydodecyl)amino]butyl}piperazine-2,5-dione (cKK-E12), bis((Z)-non-2-en-1-yl) 9-(4-(dimethylamino)butyryloxy)heptadecanedioate (L319), 2,2-dilinoleyl-4-(2-dimethylaminoethyl)-[1,3]-dioxolane (DLin-KC2-DMA), heptadec-9-yl 8-[(2-hydroxyethyl)(8-nonyloxy-8-oxooctyl)amino]octanoate (Lipid 5), 1,1'-[(2-{4-[2-({2-[bis(2-hydroxydodecyl)amino]ethyl}(2-hydroxydodecyl)amino)ethyl]piperazin-1 -yl}ethyl)azanediyl]bis(dodecan-2-ol) (C12-200), (2,3-dioleoyloxy-propyl)trimethylammonium chloride (DOTAP), dimethyldioctadecylammonium bromide (DDAB), and tetra(8-methylnonyl) 3,3',3",3‴-{[(methylazanediyl)bis(propane-3,1-diyl)]bis(azanetriyl)}tetrapropionate (306Oi10);
the phospholipid is one or more of 1,2-distearoyl-sn-glycero-3- phosphocholine (DSPC), 1,2-dioleoyl-sn-glycero-3-phosphocholine (DOPC), 1,2-dipalmitoyl-sn-glycero-3-phosphocholine (DPPC), 2-oleoyl-1-palmitoyl-sn-glycero-3-phosphocholine (POPC), 1,2-dioleoyl-sn-glycero-3-phosphoethanolamine (DOPE), 2-oleoyl-1-palmitoyl-sn-glycero-3-phosphoethanolamine (POPE), 1,2-distearoyl-sn-glycero-3-phosphoethanolamine (DSPE), and 1,2-dipalmitoyl-sn-glycero-3-phosphoethanolamine (DPPE);
the sterol compound is one or more of β-sitosterol, cholestanol, cholestanone, cholesterol, cholestenone, 7β-hydroxycholesterol, and 7α-hydroxycholesterol; and
the fatty acid is selected from a saturated fatty acid or an unsaturated fatty acid, wherein the saturated fatty acid comprises palmitic acid, stearic acid, lauric acid, myristic acid, or arachidic acid; and the unsaturated fatty acid comprises myristoleic acid, palmitoleic acid, rapeseed acid, linoleic acid, linolenic acid, oleic acid, or arachidonic acid.

5. The inhalable lipid nanoparticle for use according to any one of claims 1 to 4, wherein
the charged unit is a unit composed of at least one of the following negatively charged substances: aspartic acid-serine-serine-cysteine; a DNA of any sequence with a length of 1 to 40 bases; and formic acid; and/or
the lipid unit is a unit composed of at least one of a phospholipid, and a C₁₀-C₂₀ saturated hydrocarbon.

6. The inhalable lipid nanoparticle for use according to any one of claims 1 to 5, wherein the lipid-charged molecule conjugate is any one of a compound of formula (I), a compound of formula (II) and 2-hexyldecanoic acid,

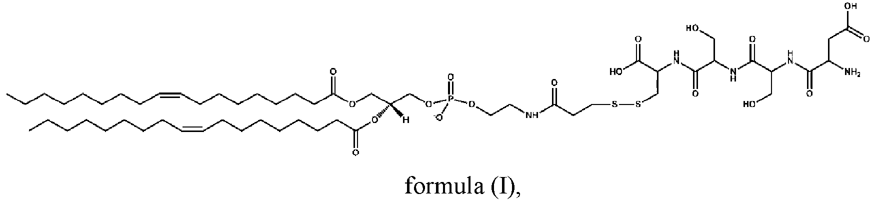

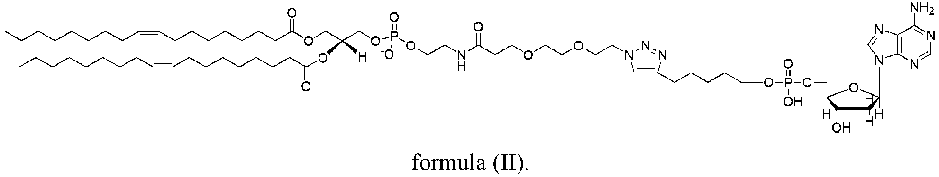

7. The inhalable lipid nanoparticle for use according to any one of claims 1 to 6, wherein the lipid components comprises an ionizable lipid, optionally a helper phospholipid, cholesterol and its derivatives, and a pegylated lipid, and the payload is a nucleic acid, wherein the nucleic acid is encapsulated in the lipid nanoparticle.

8. The inhalable lipid nanoparticle for use according to claim 7, wherein in the lipid components, the ionizable lipid is selected from the group consisting of heptadec-9-yl 8-[(2-hydroxyethyl)(6-oxo-6-undecyloxyhexyl)amino]octanoate (SM-102), [(4-hydroxybutyl)azanediyl]bis(hexane-6,1-diyl) bis(2-hexyldecanoate) (ALC-0315), (dilinoleyl) methyl 4-(N,N-dimethylamino)butyrate (DLin-MC3-DMA), 3,6-bis{4-[bis(2-hydroxydodecyl)amino]butyl}piperazine-2,5-dione (cKK-E12), bis((Z)-non-2-en-1-yl) 9-(4-(dimethylamino)butyryloxy)heptadecanedioate (L319), 2,2-dilinoleyl-4-(2-dimethylaminoethyl)-[1,3]-dioxolane (DLin-KC2-DMA), heptadec-9-yl 8-[(2-hydroxyethyl)(8-nonyloxy-8-oxooctyl)amino]octanoate (Lipid 5), 1,1'-[(2-{4-[2-({2-[bis(2-hydroxydodecyl)amino]ethyl}(2-hydroxydodecyl)amino)ethyl]piperazin-1 -yl}ethyl)azanediyl]bis(dodecan-2-ol) (C12-200), (2,3-dioleoyloxy-propyl)trimethylammonium chloride (DOTAP), dimethyldioctadecylammonium bromide (DDAB), and tetra(8-methylnonyl) 3,3',3",3‴-{[(methylazanediyl)bis(propane-3,1-diyl)]bis(azanetriyl)}tetrapropionate (306Oi10); and/or
the helper phospholipid is selected from the group consisting of 1,2-distearoyl-sn-glycero-3-phosphocholine (DSPC), 1,2-dioleoyl-sn-glycero-3-phosphocholine (DOPC), 1,2-dipalmitoyl-sn-glycero-3-phosphocholine (DPPC), 2-oleoyl-1-palmitoyl-sn-glycero-3-phosphocholine (POPC), 1,2-dioleoyl-sn-glycero-3-phosphoethanolamine (DOPE), 2-oleoyl-1-palmitoyl-sn-glycero-3-phosphoethanolamine (POPE), 1,2-distearoyl-sn-glycero-3-phosphoethanolamine (DSPE), and 1,2-dipalmitoyl-sn-glycero-3-phosphoethanolamine (DPPE); and/or
the cholesterol and its derivatives is selected from the group consisting of β-sitosterol, cholestanol, cholestanone, cholesterol, cholestenone, 7β-hydroxycholesterol, and 7α-hydroxycholesterol; and/or
the pegylated lipid is selected from the group consisting of 1,2-dimyristoyl-rac-glycero-3-methoxypolyethylene glycol (DMG-PEG), 1,2-distearoyl-rac-glycero-3-methoxypolyethylene glycol (DSG-PEG), 1,2-dipalmitoyl-rac-glycero-3-methoxypolyethylene glycol (DPG-PEG), and 1,2-distearoyl-sn-glycero-3-phosphoethanolamine-methoxypolyethylene glycol (DSPE-PEG).

9. The lipid nanoparticle for use according to claim 7 or 8, wherein in the lipid nanoparticle, the molar ratio of the lipid-charged molecule conjugate to the lipid components is (0.1 - 20):100; and/or
the ionizable lipid is 30-70 mol% with respect to the total amount of the lipid components; and/or
the helper phospholipid is 0-20 mol% with respect to the total amount of the lipid components; and/or
the cholesterol and its derivatives is 20-50 mol% with respect to the total amount of the lipid components; and/or
the pegylated lipid is 0.1-10 mol% with respect to the total amount of the lipid components;
preferably, the molar ratio of the lipid-charged molecule conjugate to the lipid components is (0.6 - 10):100; and/or
the ionizable lipid is 40-60 mol% with respect to the total amount of the lipid components; and/or
the helper phospholipid is 5-10 mol% with respect to the total amount of the lipid components; and/or
the cholesterol and its derivatives is 30-40 mol% with respect to the total amount of the lipid components; and/or
the pegylated lipid is 1-5 mol% with respect to the total amount of the lipid components.

10. The lipid nanoparticle for use according to any one of claims 7 to 9, wherein the nucleic acid is at least one of a mRNA, a small interfering ribonucleic acid, a plasmid, an antisense DNA, an aptamer, a microRNA, and a circular RNA; and/or
in the lipid nanoparticle, the nucleic acid has an encapsulation efficiency of 30 - 99%; and/or
the lipid nanoparticle has a hydrodynamic diameter of 50 - 200 nm.

11. An inhalable pharmaceutical product or biological product for use in preventing and/or treating infectious diseases transmitted through mucous membranes, mucous membrane-related tumors, or respiratory diseases, comprising the inhalable lipid nanoparticle according to any one of claims 1 to 10, wherein inhalable pharmaceutical product or biological product is administered by nebulization.

12. A lipid-charged molecule conjugate, wherein the lipid-charged molecule conjugate comprises a lipid unit and a charged unit, wherein the charged unit is a unit composed of at least one of the following negatively charged substances: aspartic acid-serine-serine-cysteine; and a DNA of any sequence with a length of 1 to 40 bases; and
the lipid unit is a unit composed of a phospholipid.

13. The lipid-charged molecule conjugate according to claim 12, wherein the lipid-charged molecule conjugate is a compound of formula (I) or a compound of formula (II),

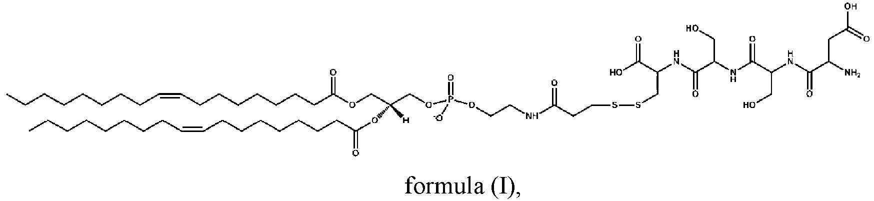

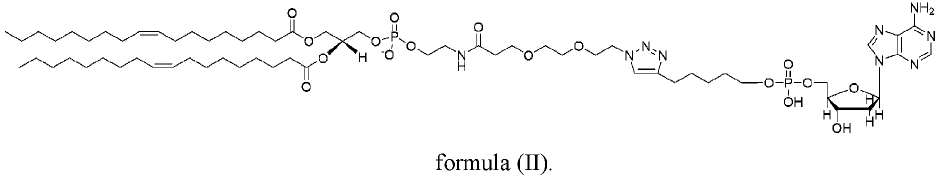

## Patentansprüche

1. Inhalierbares Lipid-Nanopartikel zur Prävention und/oder Behandlung von Infektionskrankheiten, die über Schleimhäute übertragen werden, von Schleimhauttumoren oder Atemwegserkrankungen, umfassend ein Lipid-beladenes Molekülkonjugat, Lipid-Komponenten und eine Nutzlast, wobei das Lipid-beladene Molekülkonjugat durch Selbstassemblierung mit den Lipid-Komponenten und der Nutzlast unter Einwirkung hydrophober und elektrostatischer Wechselwirkungen das Lipid-Nanopartikel bildet, wobei das Lipid-beladene Molekülkonjugat eine Lipid-Einheit und eine geladene Einheit umfasst, wobei die geladene Einheit eine negativ geladene Einheit und/oder eine positiv geladene Einheit darstellt;
wobei das inhalierbare Lipid-Nanopartikel durch Vernebelung verabreicht wird;
wobei die negativ geladene Einheit eine Einheit ist, die aus mindestens einer der folgenden negativ geladenen Substanzen besteht: einem ersten Polypeptid, einer Nukleinsäureeinheit, einem negativ geladenen Polymer und einem kleinen negativ geladenen Molekül, wobei das erste Polypeptid eine hydrophile Peptidkette ist, die eine negativ geladene Aminosäure enthält und eine Länge von 1 bis 50 Aminosäuren aufweist;
die positiv geladene Einheit eine Einheit ist, die aus mindestens einer der folgenden positiv geladenen Substanzen besteht: einem zweiten Polypeptid und einem positiv geladenen Polymer, wobei das zweite Polypeptid eine hydrophile Peptidkette ist, die eine positiv geladene Aminosäure enthält und eine Länge von 1 bis 50 Aminosäuren aufweist; und
die Lipid-Einheit eine hydrophobe Gruppe umfasst; und
wobei es sich bei der negativ geladenen Aminosäure um Asparaginsäure oder Glutaminsäure handelt; bei der Nukleinsäureeinheit sich um eine DNA oder RNA beliebiger Sequenz mit einer Länge von 1 bis 40 Basen handelt;
bei dem negativ geladenen Polymer um ein hydrophiles Polymer handelt, ausgewählt aus Hyaluronsäure, Polyacrylsäure, einem carboxylsäuremodifizierten Polyethylenglykol oder einem phosphorsäuremodifizierten Polyethylenglykol;
das kleine, negativ geladene Molekül ausgewählt ist aus einer niedermolekularen Verbindung mit einer Carbonsäuregruppe, einer niedermolekularen Verbindung mit einer Phosphorsäuregruppe, einer niedermolekularen Verbindung mit einer Sulfonsäuregruppe und einer niedermolekularen Verbindung mit einer Salpetersäuregruppe;
die positiv geladene Aminosäure ausgewählt ist aus Histidin, Arginin und Lysin;
das positiv geladene Polymer mindestens ein kationisches Polymer ist, das aus Polylysin, Chitosan und einem Poly(β-aminoester) ausgewählt ist; und
die Lipideinheit eine substituierte oder unsubstituierte gesättigte Kohlenwasserstoffgruppe oder eine Kohlenwasserstoffgruppe mit einer ungesättigten Bindung umfasst.

2. Inhalierbares Lipid-Nanopartikel zur Verwendung nach Anspruch 1, wobei das erste Polypeptid aus Asparaginsäure-Serin-Serin-Cystein ausgewählt ist; und
das kleine negativ geladene Molekül mindestens eines der Folgenden ist: 2-(3-Amino-3-oxopropyl)malonsäure, Bernsteinsäure, 2-Aminobutandisäure, Ameisensäure, Essigsäure, Propionsäure, Buttersäure, Valeriansäure, Capronsäure, Heptansäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Retinsäure oder Tamibaroten.

3. Inhalierbares Lipid-Nanopartikel zur Verwendung nach einem der Ansprüche 1 bis 2, wobei die Lipideinheit eine Einheit ist, die aus mindestens einem von einem ionisierbaren Lipid, einem Phospholipid, einer Sterolverbindung und einer Fettsäure besteht.

4. Inhalierbares Lipid-Nanopartikel zur Verwendung nach Anspruch 3, wobei
das ionisierbare Lipid mindestens eines der Folgenden ist: Heptadec-9-yl-8-[(2-hydroxyethyl)(6-oxo-6-undecyloxyhexyl)amino]octanoat (SM-102), [(4-Hydroxybutyl)azandiyl]bis(hexan-6,1-diyl)-bis(2-hexyldecanoat) (ALC-0315), (Dilinoleyl)methyl-4-(N,N-dimethylamino)butyrat (DLin-MC3-DMA), 3,6-Bis{4-[bis(2-hydroxydodecyl)amino]butyl}piperazin-2,5-dion (cKK-E12), Bis((Z)-non-2-en-1-yl)-9-(4-(dimethylamino)butyryloxy)heptadecandioat (L319), 2,2-Dilinoleyl-4-(2-dimethylaminoethyl)-[1,3]-dioxolan (DLin-KC2-DMA), Heptadec-9-yl-8-[(2-hydroxyethyl)(8-nonyloxy-8-oxooctyl)amino]octanoat (Lipid 5), 1,1'-[(2-{4-[2-({2-[bis(2-hydroxydodecyl)amino]ethyl}(2-hydroxydodecyl)amino)ethyl]piperazin-1-yl}ethyl)azandiyl]bis(dodecan-2-ol) (C12-200), (2,3-Dioleoyloxypropyl)trimethylammoniumchlorid (DOTAP), Dimethyldioctadecylammoniumbromid (DDAB) und Tetra(8-methylnonyl)-3,3',3",3‴-{[(methylazandiyl)bis(propan-3,1-diyl)]bis(azantriyl)}tetrapropionat (306Oi10);
das Phospholipid eines oder mehrere der Folgenden ist: 1,2-Distearoyl-sn-glycero-3-phosphocholin (DSPC), 1,2-Dioleoyl-sn-glycero-3-phosphocholin (DOPC), 1,2-Dipalmitoyl-sn-glycero-3-phosphocholin (DPPC), 2-Oleoyl-1-palmitoyl-sn-glycero-3-phosphocholin (POPC), 1,2-Dioleoyl-sn-glycero-3-phosphoethanolamin (DOPE), 2-Oleoyl-1-palmitoyl-sn-glycero-3-phosphoethanolamin (POPE), 1,2-Distearoyl-sn-glycero-3-phosphoethanolamin (DSPE) und 1,2-Dipalmitoyl-sn-glycero-3-phosphoethanolamin (DPPE);
die Sterolverbindung eines oder mehrere der Folgenden ist: β-Sitosterol, Cholestanol, Cholestanon, Cholesterin, Cholestenon, 7β-Hydroxycholesterol und 7α-Hydroxycholesterol; und
die Fettsäure aus einer gesättigten Fettsäure oder einer ungesättigten Fettsäure ausgewählt ist, wobei die gesättigte Fettsäure Palmitinsäure, Stearinsäure, Laurinsäure, Myristinsäure oder Arachinsäure umfasst, und die ungesättigte Fettsäure Myristoleinsäure, Palmitoleinsäure, Rapsfettsäure, Linolsäure, Linolensäure, Ölsäure oder Arachidonsäure umfasst.

5. Inhalierbares Lipid-Nanopartikel zur Verwendung nach einem der Ansprüche 1 bis 4, wobei
die geladene Einheit eine Einheit ist, die aus mindestens einer der folgenden negativ geladenen Substanzen besteht: Asparaginsäure-Serin-Serin-Cystein, eine DNA beliebiger Sequenz mit einer Länge von 1 bis 40 Basen, und Ameisensäure; und/oder
die Lipideinheit eine Einheit ist, die aus mindestens einem von Phospholipid und gesättigtem C₁₀-C₂₀-Kohlenwasserstoff besteht.

6. Inhalierbares Lipid-Nanopartikel zur Verwendung nach einem der Ansprüche 1 bis 5, wobei das Lipid-beladene Molekülkonjugat eines der Folgenden ist: eine Verbindung der Formel (I), eine Verbindung der Formel (II) und 2-Hexyldecansäure,

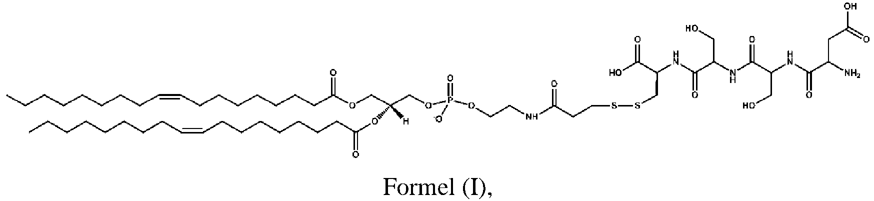

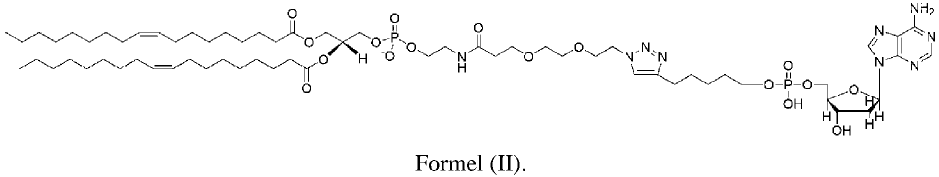

7. Inhalierbares Lipid-Nanopartikel zur Verwendung nach einem der Ansprüche 1 bis 6, wobei die Lipidkomponenten ein ionisierbares Lipid, optional ein Hilfs-Phospholipid, Cholesterin und dessen Derivate sowie ein pegyliertes Lipid umfassen und die Nutzlast eine Nukleinsäure ist, wobei die Nukleinsäure in dem Lipid-Nanopartikel verkapselt ist.

8. Inhalierbares Lipid-Nanopartikel zur Verwendung nach Anspruch 7, wobei in den Lipidkomponenten das ionisierbare Lipid aus der Gruppe ausgewählt ist, bestehend aus Heptadec-9-yl-8-[(2-hydroxyethyl)(6-oxo-6-undecyloxyhexyl)amino]octanoat (SM-102), [(4-Hydroxybutyl)azanediyl]bis(hexan-6,1-diyl)bis(2-hexyldecanoat) (ALC-0315), (Dilinoleyl)methyl-4-(N,N-dimethylamino)butyrat (DLin-MC3-DMA), 3,6-Bis{4-[bis(2-hydroxydodecyl)amino]butyl}piperazin-2,5-dion (cKK-E12), Bis((Z)-non-2-en-1-yl)-9-(4-(dimethylamino)butyryloxy)heptadecandioat (L319), 2,2-Dilinoleyl-4-(2-dimethylaminoethyl)-[1,3]-dioxolan (DLin-KC2-DMA), Heptadec-9-yl-8-[(2-hydroxyethyl)(8-nonyloxy-8-oxooctyl)amino]octanoat (Lipid 5), 1,1'-[(2-{4-[2-({2-[Bis(2-hydroxydodecyl)amino]ethyl}(2-hydroxydodecyl)amino)ethyl]piperazin-1-yl}ethyl)azanediyl]bis(dodecan-2-ol) (C12-200), (2,3-Dioleoyloxy-propyl)trimethylammoniumchlorid (DOTAP), Dimethyldioctadecylammoniumbromid (DDAB) sowie Tetra(8-methylnonyl)-3,3',3,"3"'-{ [(Methylazanediyl)bis(propan-3,1-diyl)]bis(azanetriyl)}tetrapropionat (306Oi10); und/oder
das Hilfs-Phospholipid aus der Gruppe ausgewählt ist, bestehend aus 1,2-Distearoyl-sn-glycero-3-phosphocholin (DSPC), 1,2-Dioleoyl-sn-glycero-3-phosphocholin (DOPC), 1,2-Dipalmitoyl-sn-glycero-3-phosphocholin (DPPC), 2-Oleoyl-1-palmitoyl-sn-glycero-3-phosphocholin (POPC), 1,2-Dioleoyl-sn-glycero-3-phosphoethanolamin (DOPE), 2-Oleoyl-1-palmitoyl-sn-glycero-3-phosphoethanolamin (POPE), 1,2-Distearoyl-sn-glycero-3-phosphoethanolamin (DSPE) und 1,2-Dipalmitoyl-sn-glycero-3-phosphoethanolamin (DPPE); und/oder
das Cholesterin und dessen Derivate aus der Gruppe ausgewählt sind, bestehend aus β-Sitosterol, Cholestanol, Cholestanon, Cholesterin, Cholestenon, 7β-Hydroxycholesterin und 7α-Hydroxycholesterin; und/oder
das pegylierte Lipid aus der Gruppe ausgewählt ist, bestehend aus 1,2-Dimyristoyl-rac-glycero-3-methoxypolyethylenglycol (DMG-PEG), 1,2-Distearoyl-rac-glycero-3-methoxypolyethylenglycol (DSG-PEG), 1,2-Dipalmitoyl-rac-glycero-3-methoxypolyethylenglycol (DPG-PEG) und 1,2-Distearoyl-sn-glycero-3-phosphoethanolamin-methoxypolyethylenglycol (DSPE-PEG).

9. Lipid-Nanopartikel zur Verwendung nach Anspruch 7 oder 8, wobei in dem Lipid-Nanopartikel das Molverhältnis des Lipid-beladenen Molekülkonjugats zu den Lipidkomponenten (0,1 - 20):100 beträgt; und/oder
das ionisierbare Lipid 30 - 70 Mol-% in Bezug auf die Gesamtmenge der Lipidkomponenten ausmacht; und/oder
das Hilfs-Phospholipid 0 - 20 Mol-% in Bezug auf die Gesamtmenge der Lipidkomponenten ausmacht; und/oder
das Cholesterin und dessen Derivate 20 - 50 Mol-% in Bezug auf die Gesamtmenge der Lipidkomponenten ausmachen; und/oder
das pegylierte Lipid 0,1 - 10 Mol-% in Bezug auf die Gesamtmenge der Lipidkomponenten ausmacht;
vorzugsweise das Molverhältnis des Lipid-beladenen Molekülkonjugats zu den Lipidkomponenten (0,6 - 10):100 beträgt; und/oder
das ionisierbare Lipid 40 - 60 Mol-% in Bezug auf die Gesamtmenge der Lipidkomponenten ausmacht; und/oder
das Hilfs-Phospholipid 5 - 10 Mol-% in Bezug auf die Gesamtmenge der Lipidkomponenten ausmacht; und/oder
das Cholesterin und dessen Derivate 30 - 40 Mol-% in Bezug auf die Gesamtmenge der Lipidkomponenten ausmachen; und/oder
das pegylierte Lipid 1 - 5 Mol-% in Bezug auf die Gesamtmenge der Lipidkomponenten ausmacht.

10. Lipid-Nanopartikel zur Verwendung nach einem der Ansprüche 7 bis 9, wobei die Nukleinsäure mindestens eine der Folgenden ist: eine mRNA, eine kleine interferierende Ribonukleinsäure (siRNA), ein Plasmid, eine Antisense-DNA, ein Aptamer, eine microRNA und eine zirkuläre RNA; und/oder
die Nukleinsäure in dem Lipid-Nanopartikel eine Verkapselungseffizienz von 30 - 99 % aufweist; und/oder
das Lipid-Nanopartikel einen hydrodynamischen Durchmesser von 50 - 200 nm aufweist.

11. Inhalierbares Arzneimittel oder biologisches Produkt zur Verwendung zur Prävention und/oder Behandlung von über Schleimhäute übertragenen Infektionskrankheiten, von Schleimhauttumoren oder von Atemwegserkrankungen, umfassend ein inhalierbares Lipid-Nanopartikel gemäß einem der Ansprüche 1 bis 10, wobei das inhalierbare Arzneimittel oder biologische Produkt durch Vernebelung verabreicht wird.

12. Lipid-beladenes Molekülkonjugat, wobei das Lipid-beladene Molekülkonjugat eine Lipid-Einheit und eine geladene Einheit umfasst, wobei die geladene Einheit eine Einheit darstellt, die aus mindestens einer der folgenden negativ geladenen Substanzen besteht: Asparaginsäure-Serin-Serin-Cystein; und einer DNA beliebiger Sequenz mit einer Länge von 1 bis 40 Basen; und
die Lipid-Einheit eine aus einem Phospholipid bestehende Einheit darstellt.

13. Lipid-beladenes Molekülkonjugat nach Anspruch 12, wobei es sich bei dem Lipid-beladenen Molekülkonjugat um eine Verbindung der Formel (I) oder eine Verbindung der Formel (II) handelt,

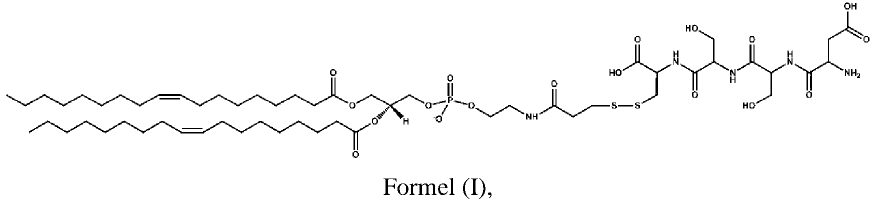

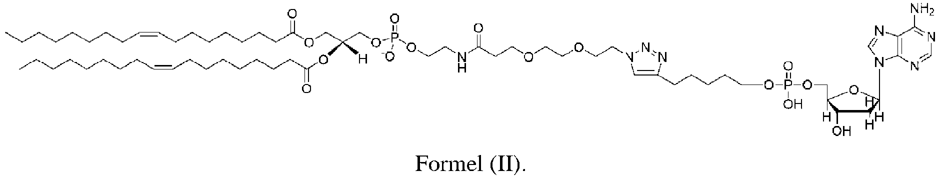

## Revendications

1. Nanoparticule lipidique inhalable destinée à une utilisation dans la prévention et/ou le traitement de maladies infectieuses transmises par les muqueuses, des tumeurs associées aux muqueuses ou de maladies respiratoires, comprenant un conjugué de molécule lipidique-chargée, des composants lipidiques et une charge utile, dans laquelle le conjugué de molécule lipidique-chargée s'autoassemblant avec les composants lipidiques et la charge utile, par une interaction hydrophobe et une interaction électrostatique, pour former la nanoparticule lipidique, dans laquelle le conjugué de molécule lipidique-chargée comprend une unité lipidique et une unité chargée, dans laquelle l'unité chargée est une unité chargée négativement et/ou une unité chargée positivement ;
dans laquelle la nanoparticule lipidique inhalable est administrée par nébulisation ;
dans laquelle l'unité chargée négativement est une unité composée d'au moins l'une parmi les substances chargées négativement suivantes : un premier polypeptide, une unité d'acide nucléique, un polymère chargé négativement et une petite molécule chargée négativement, dans laquelle le premier polypeptide est une chaîne peptidique hydrophile contenant un acide aminé chargé négativement et ayant une longueur de 1 à 50 acides aminés ;
l'unité chargée positivement est une unité composée d'au moins l'une parmi les substances chargées positivement suivantes : un deuxième polypeptide et un polymère chargé positivement, dans laquelle le deuxième polypeptide est une chaîne peptidique hydrophile contenant un acide aminé chargé positivement et ayant une longueur de 1 à 50 acides aminés ; et
l'unité lipidique comprend un groupe hydrophobe ; et
dans laquelle l'acide aminé chargé négativement est l'acide aspartique ou l'acide glutamique ;
l'unité d'acide nucléique est un ADN ou un ARN d'une quelconque séquence d'une longueur de 1 à 40 bases ;
le polymère chargé négativement est un polymère hydrophile choisi parmi l'acide hyaluronique, l'acide polyacrylique, un polyéthylène glycol modifié par acide carboxylique ou un polyéthylène glycol modifié par acide phosphorique ;
la petite molécule chargée négativement est choisie parmi un petit composé moléculaire avec un groupe acide carboxylique, un petit composé moléculaire avec un groupe acide phosphorique, un petit composé moléculaire avec un groupe acide sulfonique et un petit composé moléculaire avec un groupe acide nitrique ;
l'acide aminé chargé positivement est choisi parmi l'histidine, l'arginine et la lysine ;
le polymère chargé positivement est au moins un polymère cationique choisi parmi la polylysine, le chitosane et un poly(β-amino ester) ; et
l'unité lipidique comprend un groupe hydrocarboné saturé substitué ou non substitué ou un groupe hydrocarboné avec une liaison insaturée.

2. Nanoparticule lipidique inhalable destinée à une utilisation selon la revendication 1, dans laquelle le premier polypeptide est choisi parmi acide aspartique-sérine-sérine-cystéine ; et
la petite molécule chargée négativement est au moins l'une parmi acide 2-(3-amino-3-oxopropyl)malonique, acide succinique, acide 2-aminobutanedioïque, acide formique, acide acétique, acide propionique, acide butyrique, acide valérique, acide caproïque, acide heptanoïque, acide caprylique, acide pélargonique, acide caprique, acide undécanoïque, acide laurique, acide tridécanoïque, acide myristique, acide pentadécanoïque, acide palmitique, acide margarique, acide stéarique, acide rétinoïque ou tamibarotène.

3. Nanoparticule lipidique inhalable destinée à une utilisation selon l'une quelconque des revendications 1 à 2, dans laquelle l'unité lipidique est une unité composée d'au moins l'un parmi un lipide ionisable, un phospholipide, un composé stérolique et un acide gras.

4. Nanoparticule lipidique inhalable destinée à une utilisation selon la revendication 3, dans laquelle
le lipide ionisable est au moins l'un parmi heptadéc-9-yl 8-[(2-hydroxyéthyl)(6-oxo-6-undécyloxyhexyl)amino]octanoate (SM-102), [(4-hydroxybutyl)azanediyl]bis(hexane-6,1-diyl) bis(2-hexyldécanoate) (ALC-0315), (dilinoléyl) méthyl 4-(N,N-diméthylamino)butyrate (DLin-MC3-DMA), 3,6-bis{4-[bis(2-hydroxydodécyl)amino]butyl}pipérazine-2,5-dione (cKK-E12), bis((Z)-non-2-én-1-yl) 9-(4-(diméthylamino)butyryloxy)heptadécanedioate (L319), 2,2-dilinoléyl-4-(2-diméthylaminoéthyl)-[1,3]-dioxolane (DLin-KC2-DMA), heptadéc-9-yl 8-[(2-hydroxyéthyl)(8-nonyloxy-8-oxooctyl)amino]octanoate (Lipide 5), 1,1'-[(2-{4-[2-({2-[bis(2-hydroxydodécyl)amino]éthyl}(2-hydroxydodécyl)amino)éthyl]pipérazin-1-yl}éthyl)azanediyl]bis(dodécan-2-ol) (C12-200), chlorure de (2,3-dioleoyloxy-propyl)triméthylammonium (DOTAP), bromure de diméthyldioctadécylammonium (DDAB) et tétra(8-méthylnonyl) 3,3',3",3‴-{[(méthylazanediyl)bis(propane-3,1-diyl)]bis(azanetriyl)}tétrapropionate (306Oi10) ;
le phospholipide est un ou plusieurs parmi 1,2-distéaroyl-sn-glycéro-3-phosphocholine (DSPC), 1,2-dioleoyl-sn-glycéro-3-phosphocholine (DOPC), 1,2-dipalmitoyl-sn-glycéro-3-phosphocholine (DPPC), 2-oléoyl-1-palmitoyl-sn-glycéro-3-phosphocholine (POPC), 1,2-dioleoyl-sn-glycéro-3-phosphoéthanolamine (DOPE), 2-oléoyl-1-palmitoyl-sn-glycéro-3-phosphoéthanolamine (POPE), 1,2-distéaroyl-sn-glycéro-3-phosphoéthanolamine (DSPE) et 1,2-dipalmitoyl-sn-glycéro-3-phosphoéthanolamine (DPPE) ;
le composé stérolique est un ou plusieurs parmi β-sitostérol, cholestanol, cholestanone, cholestérol, cholesténone, 7β-hydroxycholestérol et 7α-hydroxycholestérol ; et
l'acide gras est choisi parmi un acide gras saturé ou un acide gras insaturé, dans laquelle l'acide gras saturé comprend l'acide palmitique, l'acide stéarique, l'acide laurique, l'acide myristique ou l'acide arachidique ; et l'acide gras insaturé comprend l'acide myristoléique, l'acide palmitoléique, l'acide de colza, l'acide linoléique, l'acide linolénique, l'acide oléique ou l'acide arachidonique.

5. Nanoparticule lipidique inhalable destinée à une utilisation selon l'une quelconque des revendications 1 à 4, dans laquelle
l'unité chargée est une unité composée d'au moins l'une parmi les substances chargées négativement suivantes : acide aspartique-sérine-sérine-cystéine ; un ADN d'une quelconque séquence d'une longueur de 1 à 40 bases ; et acide formique ; et/ou
l'unité lipidique est une unité composée d'au moins l'un parmi un phospholipide et un hydrocarbure saturé en C₁₀-C₂₀.

6. Nanoparticule lipidique inhalable destinée à une utilisation selon l'une quelconque des revendications 1 à 5, dans laquelle le conjugué de molécule lipidique-chargée est l'un quelconque parmi un composé de formule (I), un composé de formule (II) et l'acide 2-hexyldécanoïque,

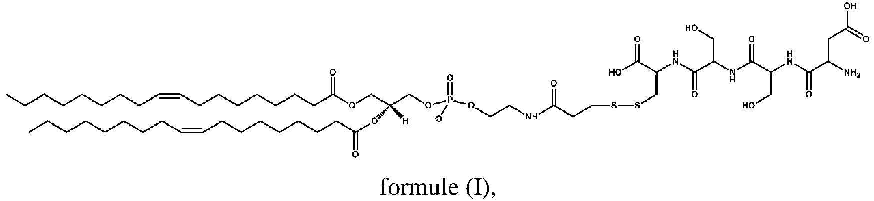

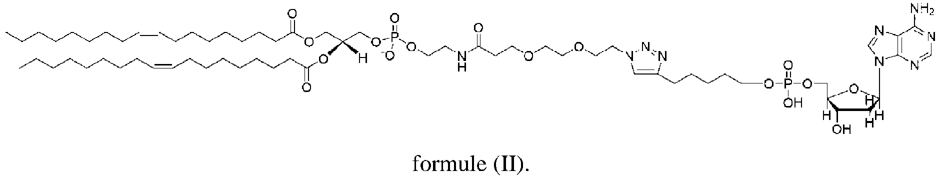

7. Nanoparticule lipidique inhalable destinée à une utilisation selon l'une quelconque des revendications 1 à 6, dans laquelle les composants lipidiques comprennent un lipide ionisable, facultativement un phospholipide auxiliaire, du cholestérol et ses dérivés, et un lipide pégylé, et la charge utile est un acide nucléique, dans laquelle l'acide nucléique est encapsulé dans la nanoparticule lipidique.

8. Nanoparticule lipidique inhalable destinée à une utilisation selon la revendication 7, dans laquelle, dans les composants lipidiques, le lipide ionisable est choisi parmi le groupe constitué de heptadéc-9-yl 8-[(2-hydroxyéthyl)(6-oxo-6-undécyloxyhexyl)amino]octanoate (SM-102), [(4-hydroxybutyl)azanediyl]bis(hexane-6,1-diyl) bis(2-hexyldécanoate) (ALC-0315), (dilinoléyl) méthyl 4-(N,N-diméthylamino)butyrate (DLin-MC3-DMA), 3,6-bis{4-[bis(2-hydroxydodécyl)amino]butyl}pipérazine-2,5-dione (cKK-E12), bis((Z)-non-2-én-1-yl) 9-(4-(diméthylamino)butyryloxy)heptadécanedioate (L319), 2,2-dilinoléyl-4-(2-diméthylaminoéthyl)-[1,3]-dioxolane (DLin-KC2-DMA), heptadéc-9-yl 8-[(2-hydroxyéthyl)(8-nonyloxy-8-oxooctyl)amino]octanoate (Lipide 5), 1,1'-[(2-{4-[2-({2-[bis(2-hydroxydodécyl)amino]éthyl}(2-hydroxydodécyl)amino)éthyl]pipérazin-1-yl}éthyl)azanediyl]bis(dodécan-2-ol) (C12-200), chlorure de (2,3-dioleoyloxy-propyl)triméthylammonium (DOTAP), bromure de diméthyldioctadécylammonium (DDAB) et tétra(8-méthylnonyl) 3,3',3",3"'-{[(méthylazanediyl)bis(propane-3,1-diyl)]bis(azanetriyl)}tétrapropionate (306Oi10) ; et/ou
le phospholipide auxiliaire est choisi dans le groupe constitué de 1,2-distéaroyl-sn-glycéro-3-phosphocholine (DSPC), 1,2-dioleoyl-sn-glycéro-3-phosphocholine (DOPC), 1,2-dipalmitoyl-sn-glycéro-3-phosphocholine (DPPC), 2-oléoyl-1-palmitoyl-sn-glycéro-3-phosphocholine (POPC), 1,2-dioleoyl-sn-glycéro-3-phosphoéthanolamine (DOPE), 2-oléoyl-1-palmitoyl-sn-glycéro-3-phosphoéthanolamine (POPE), 1,2-distéaroyl-sn-glycéro-3-phosphoéthanolamine (DSPE) et 1,2-dipalmitoyl-sn-glycéro-3-phosphoéthanolamine (DPPE) ; et/ou
le cholestérol et ses dérivés sont choisis parmi le groupe constitué de β-sitostérol, cholestanol, cholestanone, cholestérol, cholesténone, 7β-hydroxycholestérol et 7α-hydroxycholestérol ; et/ou
le lipide pégylé est choisi parmi le groupe constitué de 1,2-dimyristoyl-rac-glycéro-3-méthoxypolyéthylène glycol (DMG-PEG), 1,2-distéaroyl-rac-glycéro-3-méthoxypolyéthylène glycol (DSG-PEG), 1,2-dipalmitoyl-rac-glycéro-3-méthoxypolyéthylène glycol (DPG-PEG) et 1,2-distéaroyl-sn-glycéro-3-phosphoéthanolamine-méthoxypolyéthylène glycol (DSPE-PEG).

9. Nanoparticule lipidique destinée à une utilisation selon la revendication 7 ou 8, dans laquelle, dans la nanoparticule lipidique, le rapport molaire entre le conjugué de molécule lipidique-chargée et les composants lipidiques est de (0,1 - 20):100 ; et/ou
le lipide ionisable représente 30 à 70 % en moles par rapport à la quantité totale des composants lipidiques ; et/ou
le phospholipide auxiliaire représente 0 à 20 % en moles par rapport à la quantité totale des composants lipidiques ; et/ou
le cholestérol et ses dérivés représentent 20 à 50 % en moles par rapport à la quantité totale des composants lipidiques ; et/ou
le lipide pégylé représente 0,1 à 10 % en moles par rapport à la quantité totale des composants lipidiques ;
de préférence, le rapport molaire entre le conjugué de molécule lipidique-chargée et les composants lipidiques est de (0,6 - 10):100 ; et/ou
le lipide ionisable représente 40 à 60 % en moles par rapport à la quantité totale des composants lipidiques ; et/ou
le phospholipide auxiliaire représente 5 à 10 % en moles par rapport à la quantité totale des composants lipidiques ; et/ou
le cholestérol et ses dérivés représentent 30 à 40 % en moles par rapport à la quantité totale des composants lipidiques ; et/ou
le lipide pégylé représente 1 à 5 % en moles par rapport à la quantité totale des composants lipidiques.

10. Nanoparticule lipidique destinée à une utilisation selon l'une quelconque des revendications 7 à 9, dans laquelle l'acide nucléique est au moins l'un parmi un ARNm, un petit acide ribonucléique interférent, un plasmide, un ADN antisens, un aptamère, un microARN et un ARN circulaire ; et/ou dans la nanoparticule lipidique, l'acide nucléique présente un rendement d'encapsulation de 30 à 99 % ; et/ou
la nanoparticule lipidique présente un diamètre hydrodynamique de 50 à 200 nm.

11. Produit pharmaceutique ou produit biologique inhalable destiné à une utilisation dans la prévention et/ou le traitement de maladies infectieuses transmises par les muqueuses, des tumeurs associées aux muqueuses ou de maladies respiratoires, comprenant la nanoparticule lipidique inhalable selon l'une quelconque des revendications 1 à 10, dans lequel le produit pharmaceutique ou produit biologique inhalable est administré par nébulisation.

12. Conjugué de molécule lipidique-chargée, dans lequel le conjugué de molécule lipidique-chargée comprend une unité lipidique et une unité chargée, dans lequel l'unité chargée est une unité composée d'au moins l'une parmi les substances chargées négativement suivantes : acide aspartique-sérine-sérine-cystéine ; et un ADN d'une quelconque séquence d'une longueur de 1 à 40 bases ; et
l'unité lipidique est une unité composée d'un phospholipide.

13. Conjugué de molécule lipidique-chargée selon la revendication 12, dans lequel le conjugué de molécule lipidique-chargée est un composé de formule (I) ou un composé de formule (II),
